# EUROPEAN PATENT APPLICATION

(11) **EP 4 772 512 A1**
(43) Date of publication of application: **08.07.2026**
(21) Application number: 23950028.3
(22) Date of filing: 28.08.2023
(51) Int. Cl.: C07D 491/052, A61K 31/4166, A61K 31/353, A61K 47/69, A61K 9/127, A61P 25/00

(54) **DRUG FOR PREVENTING AND/OR TREATING ALZHEIMER'S DISEASE**

(71) Applicant: Shenzhen University of Advanced Technology, Shenzhen, Guangdong 518107 (CN)
(72) Inventor: YE, Keqiang, Shenzhen, Guangdong 518051 (CN)
(74) Representative: Fabry, Bernd
(86) International application number: PCT/CN2023/115202
(87) International publication number: WO 2025/043434

(57) **Abstract**

The present disclosure discloses drugs for preventing and/or treating Alzheimer's disease (AD). A CF3CN derivative provided by present disclosure has any one of structural formulas 1-4 shown below. All four CF3CN derivatives have TrkB agonist activities; and specifically, the CF3CN derivative shown in formula 2 serves as an optimal derivative. *In vivo* PK studies reveal that the CF3CN derivative shown in the formula 2 is capable of improving a B/P Ratio of CF3CN, and overcoming the limitations of CF3CN. Nanoparticles are prepared by encapsulating the CF3CN derivatives with zein and lactoferrin, which may further enhance an oral bioavailability and a brain drug concentration, thereby improving AD treatment effects. By further improving the formulation and administration route, a liposome is employed to encapsulate the CF3CN derivative for both oral and intranasal administration, which effectively solves the problems of low bioavailability and low brain drug concentration of the derivative.

## Description

### CROSS-REFERENCE TO RELATED APPLICATIONS

This application is a continuation of International Patent Application No. PCT/CN2023/115202, filed on August 28, 2023. The content of the aforementioned application, including any intervening amendments thereto, is incorporated herein by reference in its entirety.

### TECHNICAL FIELD

The present disclosure belongs to the technical field of biological medicine, and particularly relates to drugs for preventing and/or treating Alzheimer's disease.

### BACKGROUND

Alzheimer's disease (AD), commonly known as senile dementia, is a globally prevalent neurodegenerative disease. Typical pathological features of the AD include extracellular deposition of β-amyloid proteins (Aβ), and hyperphosphorylation and truncation of microtubule-associated proteins (Tau) in neurons leading to neurofibrillary tangles, which are concurrently accompanied by progressive memory and cognitive dysfunction. Currently, the U.S. Food and Drug Administration (FDA) has approved only five drugs for treating AD, and the five drugs include four acetylcholinesterase inhibitors and one glutamate receptor antagonist (memantine). However, these drugs merely alleviate symptoms temporarily and neither cure nor retard the pathological progression. A brain-derived neurotrophic factor (BDNF), as a main member of a neurotrophin family, is the most abundant neurotrophic factor in the brain. The BDNF exerts biological functions mainly by specifically binding to a high-affinity tyrosine kinase receptor B (TrkB) to activate a tyrosine protein kinase and further activate three main downstream signaling pathways: PI3K/Akt, Ras/ERK, and PLCγ1/PKC. The BDNF promotes survival, growth, and differentiation of developing neurons, regulates synaptic transmission and synaptic plasticity in a plurality of brain regions of an adult central nervous system, and ameliorates a cognitive function impaired due to memory recovery. In AD patients, levels of both BDNF mRNA and proteins are severely reduced in a plurality of brain regions including the hippocampus, temporal cortex, and frontal cortex. Upregulation of BDNF expression by an ERK/CREB signaling pathway ameliorates Aβ-induced neuronal loss and dendritic atrophy. Upon stimulation of neuronal cells by the BDNF, activation of TrkB and PI3K signaling pathways causes Tau dephosphorylation. Therefore, the BDNF/TrkB signaling pathway may serve as a therapeutic target for treating AD.

To seek a small-molecule TrkB agonist, the applicant previously performed high-throughput screening of compound libraries and discovered that 7,8-dihydroxyflavone (7,8-DHF) was a specific TrkB receptor agonist capable of simulating physiological functions of the BDNF *in vivo.* However, 7,8-DHF, a flavonoid compound, is prone to phase II metabolism *in vivo,* thereby resulting in a low oral bioavailability (approximately 4.6%). Therefore, the applicant performed lead compound optimization through chemical structural modification, and synthesized a derivative CF3CN of 7,8-DHF (Chen, C.; Ahn, E. H.; Liu, X.; Wang, Z.-H.; Luo, S.; Liao, J.; Ye, K. Optimized TrkB agonist ameliorates Alzheimer's disease pathologies and improves cognitive functions via inhibiting delta-secretase. ACS Chemical Neuroscience 2021, 12(13), 2448-2461). *In vitro* experiments confirm that CF3CN tightly binds with an LCM/CC2 motif within an extracellular domain of the TrkB, thereby activating the TrkB signaling pathway. *In vivo* animal pharmacokinetic (PK) experiments demonstrate that CF3CN exhibits a superior oral bioavailability and *in vivo* half-life. TrkB receptors and downstream signaling pathways in brains are effectively activated through oral administration of CF3CN to 5XFAD mice, which ameliorates AD symptoms in a dose-dependent manner. Additionally, CF3CN significantly enhances learning and memory functions of 3xTg mice. Although CF3CN exhibits a brain permeability, a Brain/Plasma Ratio (B/P Ratio) of CF3CN is unsatisfactory, which is approximately 1%.

Delivery of compounds by nanocarriers is an effective way to significantly enhance the oral bioavailability of poorly absorbable compounds. Nanoparticle encapsulation of compounds enhances a gastrointestinal stability of the compounds while facilitating absorption to improve the oral bioavailability. Zein, a hydrophobic protein derived from corn seeds, is readily soluble in ethanol and other organic solvents. In addition to being classified as a Generally Recognized As Safe (GRAS) material, zein is further approved by the FDA as a pharmaceutical excipient. The hydrophobicity, biodegradability, and biocompatibility characteristics of the zein enable versatile applications across multiple fields, including food coatings and encapsulation, pharmaceutical coatings, drug delivery platforms, and tissue engineering applications. The zein is prone to self-assembly into nanoparticles in different structures under different solvent and treatment conditions. Further, due to the presence of abundant hydroxyl, amino, and carboxyl groups on side chains, zein molecules enable chemical conjugation with compounds by adding crosslinking agents. Some studies have demonstrated that the zein is capable of encapsulating some hydrophobic compounds including curcumin, resveratrol, folic acid, atorvastatin, and daidzein, so as to enhance the oral bioavailability. However, zein-based nanoparticles are prone to aggregation or precipitation when exposed to high ionic strength, high temperatures, and pH conditions proximate to an isoelectric point, thereby restricting the applicability. To solve this problem, common and effective methods involve stabilizing zein nanoparticles using surface active agents or biopolymers; for example, representative biopolymers include pectin, chitosan, and caseinate. Studies have reported that lactoferrin (LF), also referred to as lactotransferrin, is a mammalian iron-binding glycoprotein with a molecular weight of 80 kDa, and functions as a stabilizing agent for nanoparticles. Additionally, transferrin receptors (TfR) positioned on intestinal epithelial cells are capable of facilitating absorption of nanoparticles by the intestinal epithelial cells. Consequently, preparing nanoparticles from zein and stabilizing agents for compound delivery is also a common method to enhance the oral bioavailability.

A liposome is a promising nanoparticle delivery formulation that meets most requirements for an ideal nanocarrier, due to the superior biocompatibility, safety, and biodegradability. The liposome has a well-defined structure, is easy to prepare, and exhibits numerous physicochemical properties. The liposome is capable of encapsulating substantially all kinds of molecules, including ipophilic, hydrophilic, and polypeptide-based drugs. The liposome is a biocompatible carrier used for enhancing the oral bioavailability of drugs. Owing to the structural similarity in bilayer, the liposome exhibits better adherence to a biological membrane. Studies have also indicated that similar to other lipid-based formulations, the liposome and bile salts may form mixed micellar structures that increase the solubility of poorly soluble drugs and facilitate lymphatic absorption. The liposome as an oral drug carrier also has certain limitations. A main deficiency of the oral liposome concerns the instability under gastrointestinal conditions, including pH variations, as well as presence of bile salts and enzymes. Another drawback is a limited capability to diffuse through a mucus layer. Fortunately, studies have proposed solutions to these problems by modifying liposome surfaces with various stabilizing agents such as PEG, chitosan, and polypeptides. These surface modification materials enhance the stability of the liposome for oral administration.

Intranasal administration is not a novel method for delivering drugs to the systemic circulation, but the novelty of the intranasal administration resides in utilizing this non-invasive approach to achieve direct and rapid drug delivery from the nasal mucosa to the brain and spinal cord, with the aim of treating central nervous system diseases while minimizing systemic exposure. Although a precise mechanism of the intranasal administration to a central nervous system is not yet fully defined, more and more evidence indicates that drug absorption into the brain following the intranasal administration mainly relies on three pathways: an olfactory nerve pathway, a trigeminal nerve pathway, and a blood circulation pathway. The first two pathways involve drug delivery along neural pathways and direct absorption into the brain. The last pathway involves drug absorption into blood circulation through abundant submucosal capillaries and lymphatic vessels, followed by penetration of a blood-brain barrier (BBB) to reach the brain. The intranasal administration mainly has the following advantages: bypassing of enterohepatic circulation avoids first-pass effects associated with oral administration, and prevents drug-induced hepatotoxicity; a lower activity of hydrolytic enzymes in the nasal mucosa compared to hydrolytic enzymes in a gastrointestinal tract typically enables effective dosing at 1/10 to 1/15 of an oral dose, making it particularly suitable for drugs susceptible to gastrointestinal degradation, such as polypeptides, hormones, and vaccines; and the intranasal administration is particularly suitable for drugs targeting the brain, and drugs are directly absorbed into the brain without penetrating the BBB, thereby enhancing brain drug concentrations.

### SUMMARY

The BDNF/TrkB signaling pathway plays a critical role in neural plasticity and neuronal survival, but is frequently deficient in brains of AD patients. Accordingly, the BDNF/TrkB signaling pathway represents a hotspot therapeutic target for AD drug development. The small-molecule compound CF3CN previously developed by the applicant may serve as a specific TrkB agonist, and plays a therapeutic role in AD mouse models, but a Brain/Plasma Ratio (B/P Ratio) of CF3CN is unsatisfactory, which is approximately 1%. To solve the problems in the prior art, the present disclosure aims to provide drugs for preventing and/or treating AD.

Prior studies of the applicant on drug structure-activity relationship (SAR) demonstrate that an imidazole derivative containing a CF3CN-bearing A-ring scaffold retains a TrkB agonist activity, and an electron-withdrawing cyano group (CN) on a B-ring contributes to enhancing the stability in both plasma and hepatic microsomes. Therefore, the applicant only modifies the CN group while maintaining an integrity of the remaining structure. In the present disclosure, different derivatives #1-4 of CF3CN are synthesized by reducing the CN groups to alkylamines and amides to enhance the B/P Ratio. The nanotechnology is further employed for compound delivery to enhance the oral bioavailability.

The specific technical solutions are as follows:
In a first aspect, the present disclosure provides a CF3CN derivative, where the CF3CN derivative has any one of structural formulas 1-4 shown below:

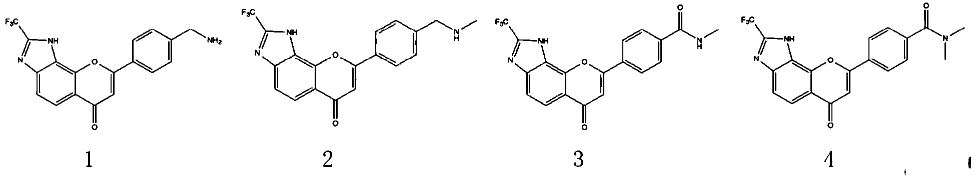

In a second aspect, the present disclosure provides a pharmaceutical composition, where the pharmaceutical composition includes one or more of CF3CN derivatives shown in formulas 1-4 below, and a pharmaceutically acceptable carrier or excipient:

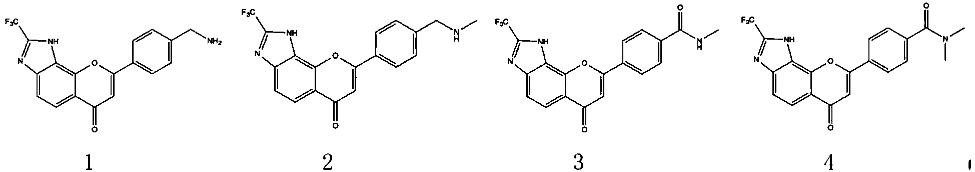

In a third aspect, the present disclosure provides a nanoparticle, where the nanoparticle includes one or more of CF3CN derivatives shown in formulas 1-4 below, and zein, and the nanoparticle is prepared by an antisolvent co-precipitation method:

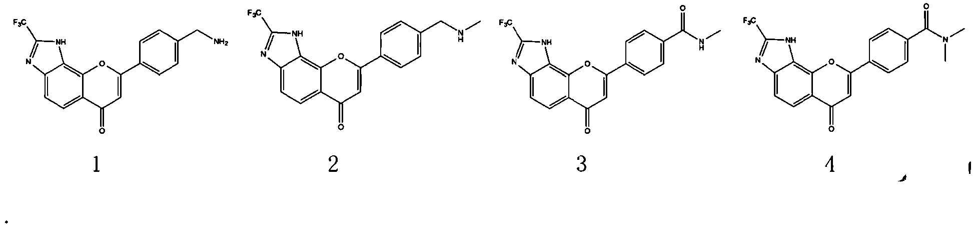

Further, the nanoparticle further includes lactoferrin or glycosylated lactoferrin, and preferably lactoferrin.

Further, a weight ratio of the CF3CN derivative to the zein is 1:1-1:20, and preferably 1:10.

Further, a weight ratio of the CF3CN derivative to the zein to the lactoferrin or glycosylated lactoferrin is (1-2):(1-20):(1-20), and preferably 1:10:10.

Further, the nanoparticle includes a CF3CN derivative shown in the formula 2, zein, and lactoferrin, and a weight ratio of the CF3CN derivative shown in the formula 2 to the zein to the lactoferrin is (1-2):(1-20):(1-20), and preferably 1:10:10.

When the nanoparticle includes one or more of CF3CN derivatives shown in the formulas 1-4 below, and zein, the nanoparticle is prepared by the antisolvent co-precipitation method including: dissolving one or more of the CF3CN derivatives shown in the formulas 1-4 and zein in a 70 vol% to 80 vol% aqueous ethanol solution to obtain a mixed solution, adding the mixed solution to deionized water under stirring, and evaporating the solvent to obtain the nanoparticle.

When the nanoparticle includes one or more of CF3CN derivatives shown in the formulas 1-4 below, zein, and lactoferrin, the nanoparticle is prepared by the antisolvent co-precipitation method including: dissolving one or more of the CF3CN derivatives shown in the formulas 1-4, zein, and lactoferrin in a 70 vol% to 80 vol% aqueous ethanol solution to obtain a mixed solution, adding the mixed solution to deionized water under stirring, and evaporating the solvent to obtain the nanoparticle.

When the nanoparticle includes one or more of CF3CN derivatives shown in the formulas 1-4 below, zein, and glycosylated lactoferrin, the nanoparticle is prepared by the antisolvent co-precipitation method including: dissolving one or more of the CF3CN derivatives shown in the formulas 1-4 and zein in a 70 vol% to 80 vol% aqueous ethanol solution to obtain a mixed solution; dissolving glycosylated lactoferrin in an aqueous solution to obtain an aqueous glycosylated lactoferrin solution, and adding the mixed solution to the aqueous glycosylated lactoferrin solution under stirring, and evaporating the solvent to obtain the nanoparticle.

In a fourth aspect, the present disclosure provides a liposome, where the liposome includes one or more of CF3CN derivatives shown in formulas 1-4 below, and lecithin, and the liposome is prepared by an ethanol injection method:

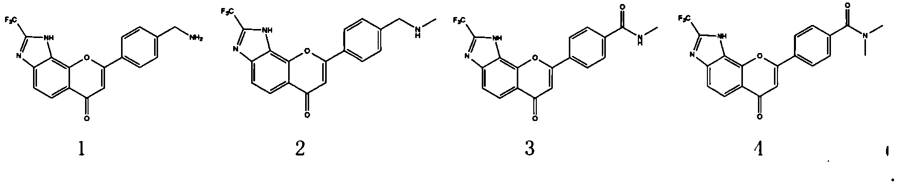

Preferably, a weight ratio of the lecithin to the CF3CN derivative is (1:1)-(50:1), and preferably 20:1.

Further, the liposome further includes a stabilizing agent;
preferably, the stabilizing agent is selected from chitosan; and
preferably, a weight ratio of the lecithin to the chitosan to the CF3CN derivative is (1:1:1)-(50:1:1), and more preferably 20:2:1.

Preferably, the liposome includes a CF3CN derivative shown in the formula 2, lecithin, and chitosan.

Further, the liposome is prepared by the ethanol injection method including: dissolving one or more of CF3CN derivatives shown in the formulas 1-4 and lecithin in absolute ethyl alcohol to obtain a mixed solution, adding the mixed solution to deionized water under stirring, and evaporating the solvent to obtain a crude liposome solution.

Preferably, the method further includes: subjecting the crude liposome solution to ice bath ultrasonication for 5-10 min; and dissolving chitosan in deionized water to obtain an aqueous chitosan solution, and adding the liposome solution to the aqueous chitosan solution under stirring to obtain the derivative-loaded liposome.

In a fifth aspect, the present disclosure provides applications of the CF3CN derivative in preparing a TrkB agonist, or applications of the CF3CN derivative, the pharmaceutical composition, the nanoparticle, or the liposome in preparing drugs for preventing and/or treating AD.

In a sixth aspect, the present disclosure provides a method for preventing and/or treating AD, where the method includes: administering to a subject a therapeutically effective amount of the above CF3CN derivative, the above pharmaceutical composition, the above nanoparticle, or the above liposome.

Further, an administration route is oral administration or intranasal administration. Preferably, an optimal administration route of the CF3CN derivative shown in the formula 2 is intranasal administration in a liposome form.

The present disclosure has the following beneficial effects:
(1) All four CF3CN derivatives #1-4 provided by the present disclosure have a TrkB agonist activity, and are used for preparing drugs for preventing and/or treating AD.
(2) The CF3CN derivative #2 provided by the present disclosure exhibits a favorable stability in hepatic microsomes, and binds with minimal plasma proteins, such that the CF3CN derivative #2 is the best derivative in the present disclosure. *In vivo* PK studies demonstrate that the B/P Ratio of CF3CN is improved, and the limitations of CF3CN are overcome.
(3) In the present disclosure, when the drugs for preventing and/or treating AD are prepared, the nanaparticle containing any one of the CF3CN derivatives is prepared from zein, and lactoferrin (LF) or deglycosylated lactoferrin (DLF) to enhance the bioavailability of the drugs. #2-NP prepared by zein/LF nanoparticle encapsulation of a compound #2 enhances the oral bioavailability and brain drug concentration, and improves effects of the compound in PK/pharmacodynamic (PD) and pharmacological experiments, which finally facilitates AD treatment effects.
(4) #2-LIP prepared by liposome encapsulation of the compound #2 enhances the oral bioavailability and brain drug concentration, and improves effects of the compound in the PK experiment.

### BRIEF DESCRIPTION OF THE DRAWINGS

FIG. 1a illustrates chemical structures of CF3CN and four reduction derivatives thereof.
FIG. 1b illustrates EC50 values of CF3CN and derivatives thereof. BR6 and T48 cells are treated with staurosporine (STS) (100 nM) and TrkB agonists. CF3CN and compounds #1-4 are added in a dose-dependent manner at concentrations of 0, 5, 10, 50, 100, 500, and 1,000 nM. After 24 h, an MTT assay is performed, and EC50 values are calculated using Prism software (n = 3; data are represented by mean + standard deviation (SD)).
FIG. 1c illustrates immunoblotting analysis of cell lysates harvested by lysis of the cells after 24 h treatment, using various antibodies.

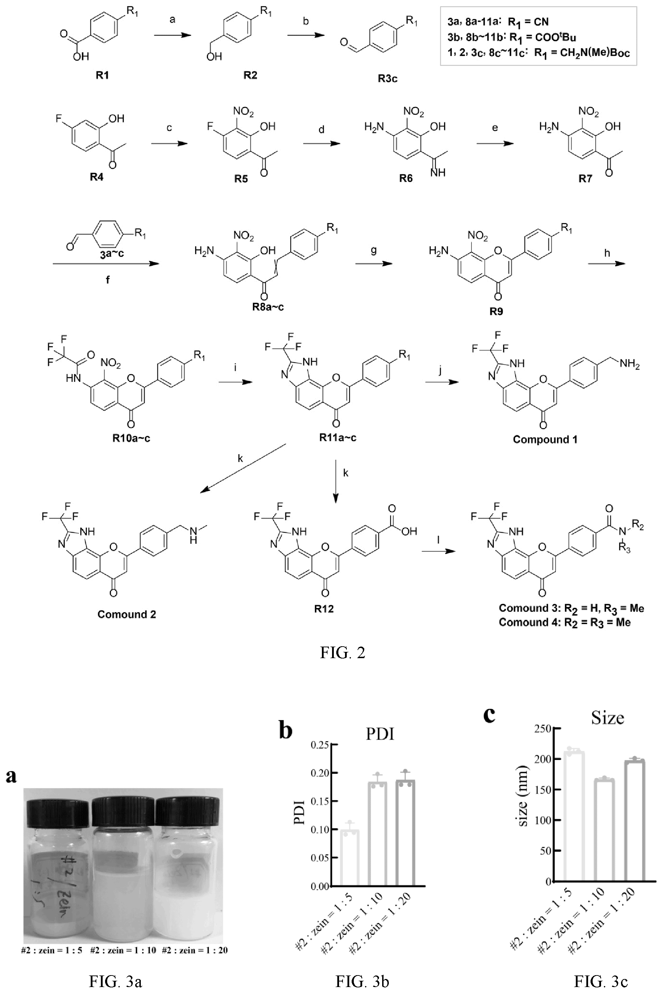

FIG. 2 illustrates an organic synthesis route of compounds # 1-4. Reagents and conditions are as follows: a involves borane-tetrahydrofuran complex (BH3/THF), which is added at 0°C, followed by reaction at room temperature; b involves Dess-Martin periodinane (DMP), NaHCO₃, and dichloromethane (DCM), which are added at 0°C, followed by reaction at room temperature; c involves HNO₄ and H₂SO₄, which are allowed for reaction at 0°C; d involves concentrated NH₄OH and dimethyl sulfoxide (DMSO), which are allowed for reaction at 50°C; e involves 1 mol/L hydrochloric acid (1N HCl) and acetonitrile (ACN), which are allowed for reaction at 90°C; f involves NaH and N,N-dimethylformamide (DMF), which are added at 0°C, followed by reaction at room temperature; g involves iodine (I₂) and DMSO, which are allowed for reaction at 130°C; h involves trifluoroacetic anhydride (TFAA) and pyridine, which are added at 0°C, followed by reaction at room temperature; i involves Fe, AcOH, and MeOH, which are allowed for reaction at 50°C; j (for preparation of R11a) involves H₂, raney-nickel catalyst (Ra-Ni), and DMF, which are allowed for reaction at 50°C; k (for preparation of R11b and R11c) involves trifluoroacetic acid (TFA) and DCM, which are allowed for reaction at 25°C; and 1 involves MeNH₂ (for preparation of compound 3); Me₂NH (for preparation of compound 4); 2-(7-azabenzotriazol-1-yl)-N,N,N',N'-tetramethyluronium hexafluorophosphate (HATU), N,N-diisopropylethylamine (DIEA), and DMF, which are added at 0°C, followed by reaction at room temperature.
FIG. 3a illustrates appearances of three nanoparticle formulations.
FIG. 3b illustrates polydispersity indexes (PDI) of three nanoparticle formulations.
FIG. 3c illustrates particle sizes of three nanoparticle formulations.
FIG. 3d illustrates size distribution by intensity of three nanoparticle formulations measured by dynamic light scattering (DLS).
FIG. 3e illustrates turbidities of three nanoparticle formulations.
FIG. 3f illustrates zeta potentials of three nanoparticle formulations.
FIG. 3g illustrates zeta potential distribution of three nanoparticle formulations measured by DLS.
FIG. 3h illustrates encapsulation efficiencies (EE) of three nanoparticle formulations.
FIG. 3i illustrates drug loading capacities (LC) of three nanoparticle formulations.
FIG. 3j illustrates a high-performance liquid chromatography (HPLC) standard curve of a compound #2 (n = 3; data are represented by mean ± SD).
FIG. 4a is an structural schematic diagram illustrating a formation mechanism of zein/LF nanoparticles.
FIG. 4b illustrates appearances of four nanoparticle formulations.
FIG. 4c illustrates PDIs of four nanoparticle formulations.
FIG. 4d illustrates particle sizes of four nanoparticle formulations.
FIG. 4e illustrates size distribution by intensity of four nanoparticle formulations measured by DLS.
FIG. 4f illustrates depict turbidities of four nanoparticle formulations.
FIG. 4g illustrates zeta potentials of four nanoparticle formulations.
FIG. 4h illustrates zeta potential distribution of four nanoparticle formulations measured by DLS (n = 3; data are represented by mean ± SD).
FIG. 5a illustrates preparation processes of #2/zein, #2/zein/LF, and #2/zein/DLF nanoparticle formulations.
FIG. 5b illustrates particle sizes of three nanoparticle formulations;
FIG. 5c illustrates zeta potentials of three nanoparticle formulations.
FIG. 5d illustrates PDIs of three nanoparticle formulations.
FIG. 5e illustrates turbidities of three nanoparticle formulations.
FIG. 5f illustrates EEs of three nanoparticle formulations.
FIG. 5g illustrates LCs of three nanoparticle formulations (n = 3; data are represented by mean ± SD).
FIG. 5h is SEM images captured at magnifications of ×40,000-80,000.
FIG. 5i is transmission electron microscopy (TEM) images of three nanoparticle formulations, with a scale bar = 500 nm.

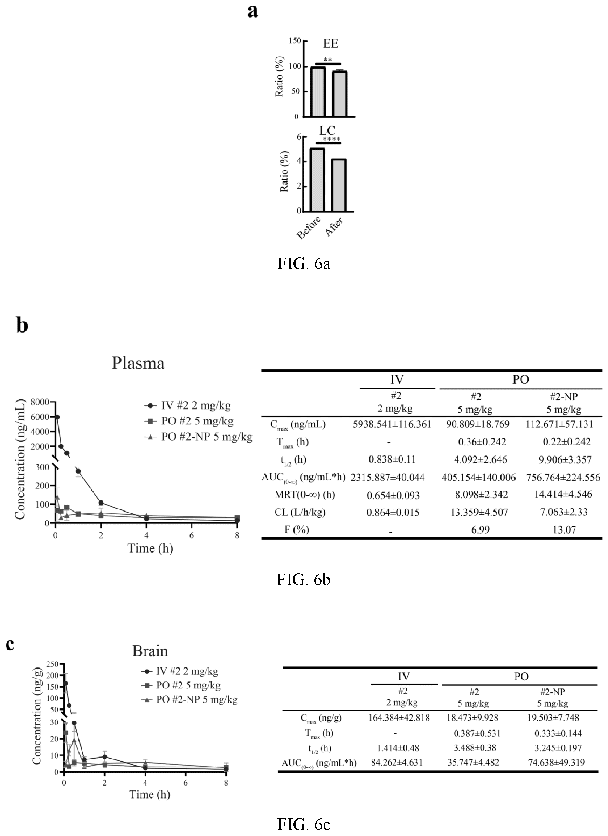

FIG. 6a illustrates EEs and LCs of #2-NP before and after lyophilization (n = 3, Student's t-test). FIG. 6b illustrates plasma concentrations following administration of #2 or #2-NP. ICR mice are divided into four groups, which are administered with #2 at 2 mg/kg (intravenous injection, IV), #2 at 5 mg/kg (oral administration, PO), #2-NP at 5 mg/kg (PO), and #2-NP at 10 mg/kg (PO). Blood samples are collected from all three mice of each group at corresponding time points, and concentrations of #2 are quantitatively analyzed by a liquid chromatography-tandem mass spectrometry (LC-MS/MS). PK parameters of each group are calculated using DAS 2.0 software, as shown in the table. Cₘₐₓ: maximum plasma concentration; Tₘₐₓ: time to maximum concentration; T½: terminal elimination half-life; AUC: area under curves; MRT: mean residence time; CL: clearance; and F: relative oral bioavailability.
FIG. 6c illustrates brain concentrations following administration of #2 or #2-NP.
FIG. 6d illustrates comparison of plasma concentrations of #2 and #2-NP after oral gavage (IG) administration at identical doses of 5 mg/kg (data are represented by mean ± SD; **p≤ 0.01, ****p≤0.0001).
FIG. 6e illustrates comparison of brain concentrations of #2 and #2-NP after oral gavage (IG) administration at identical doses of 5 mg/kg (data are represented by mean ± SD; **p≤0.01, ****p≤0.0001).
FIG. 7a illustrates plasma and brain concentrations of #2 quantitatively analyzed by LC-MS/MS after IV administration of #2 at 2 mg/kg.
FIG. 7b illustrates plasma and brain concentrations of #2 quantitatively analyzed by LC-MS/MS after IG administration of #2 at 5 mg/kg.
FIG. 7c illustrates plasma and brain concentrations of #2-NP quantitatively analyzed by LC-MS/MS after IG administration of #2-NP at 5 mg/kg.
FIG. 7d illustrates comparison of plasma drug concentrations after IV or PO administration of #2 (n = 3; data are represented by mean ± SD).
FIG. 7e illustrates comparison of brain drug concentrations after IV or PO administration of #2 (n = 3; data are represented by mean ± SD).
FIG. 7f illustrates comparison of B/P ratios of #2 and #2-NP after PO administration at identical doses of 5 mg/kg (n = 3, Student's t-test).
FIG. 8a illustrates analysis of asparaginyl endopeptidase (AEP) activities in brain lysates of APP/PS1 mice at different time points after PO administration of #2-NP. Plasma and brain concentrations of #2 are quantitatively analyzed by LC-MS/MS (n = 3; one-way ANOVA).
FIG. 8b illustrates TrkB signaling pathways and AEP expressions obtained by immunoblotting analysis on lysed brain tissues of APP/PS1 mice.
FIG. 8c illustrates result quantification of FIG. 8b (n = 3, one-way ANOVA).
FIG. 8d illustrates AEP activities in brain lysates of Tau P301S mice at different time points after PO administration of #2-NP. Plasma and brain concentrations of #2 are quantitatively analyzed by LC-MS/MS (n = 3; one-way ANOVA).
FIG. 8e illustrates TrkB signaling pathways and AEP expressions obtained by immunoblotting analysis on lysed brain tissues of Tau P301S mice.
FIG. 8f illustrates result quantification of FIG. 8e (n = 3, one-way ANOVA).
FIG. 8g illustrates acute administration effects of #2-NP on 3xTg mice. 3xTg mice are administered with #2-NP at 5 mg/kg or 10 mg/kg (PO), and blood and brain tissue samples are collected from three mice of each group after 2 h of acute treatment. Concentrations of #2 are quantitatively analyzed by LC-MS/MS.
FIG. 8h illustrates AEP activities in brain lysates of 3xTg mice treated by PO administration of #2-NP at different doses.
FIG. 8i illustrates TrkB signaling pathways and AEP expressions obtained by immunoblotting analysis on lysed brain tissues of 3xTg mice, with APP and Tau proteins cleaved.
FIG. 8j illustrates result quantification of FIG. 8i (n = 3, one-way ANOVA) (data are represented by mean ± SD, *p<0.05).
FIG. 9a illustrates representative movement trajectories in a novel object recognition (NOR) test for 3xTg mice from a Vehicle group, a #2 3 mg/kg group, a #2-NP 3 mg/kg group, and a #2-NP 10 mg/kg group.
FIG. 9b illustrates statistical analysis of recognition indexes of different groups (n = 6, one-way ANOVA).
FIG. 9c illustrates representative T2-weighted magnetic resonance imaging (MRI) scanning images of 3xTg mice, with cortical regions of interest (ROI) marked by red lines.
FIG. 9d illustrates a quantified cerebral cortex volume of ROI (n = 3, one-way ANOVA).
FIG. 9e illustrates immunohistochemical (IHC) staining of Aβ in brain sections of 3xTg mice, with a scale bar = 50 µm.
FIG. 9f illustrates a quantitative diagram of FIG. 9e (n = 6, one-way ANOVA).
FIG. 9g is [¹⁸F]AV-45 positron emission tomography-computed tomography (PET-CT) scanning images of 3xTg mice from the Vehicle group, #2 3 mg/kg group, #2-NP 3 mg/kg group, and #2-NP 10 mg/kg group.
FIG. 9h illustrates comparison of [¹⁸F]AV-45 PET standardized uptake value ratios (SUVR) per gram of the cerebral cortex of mice from different groups (n = 3, one-way ANOVA).
FIG. 9i illustrates quantitative analysis of Aβ42 in brain tissues by an enzyme-linked immunosorbent assay (ELISA) (n = 3, one-way ANOVA) (data are represented by mean ± SD; ** p < 0.01, ** p < 0.01, *** p < 0.005, ****p ≤ 0.0001).
FIG. 9j illustrates quantitative analysis of Aβ40 in brain tissues by the ELISA (n = 3, one-way ANOVA) (data are represented by mean ± SD; ** p < 0.01, ** p < 0.01, *** p < 0.005, ****p ≤ 0.0001).
FIG. 10a illustrates TrkB signaling pathways, AEP expressions, and expressions and cleavages of APP and Tau in brain tissues detected by immunoblotting.
FIG. 10b illustrates an assay of AEP activities in brain tissues (n = 6, one-way ANOVA).
FIG. 10c illustrates quantitative analysis of p-TrkB/TrkB immunoblotting results (n = 6, one-way ANOVA) (data are represented by mean ± SD; ns represents no statistically significant difference, *p < 0.05, ** p < 0.01, *** p < 0.005).
FIG. 10d illustrates quantitative analysis of p-PLCγ1/ PLCγ1 immunoblotting results (n = 6, one-way ANOVA) (data are represented by mean ± SD; ns represents no statistically significant difference, *p < 0.05, ** p < 0.01, *** p < 0.005).
FIG. 10e illustrates quantitative analysis of p-AEP/β-actin immunoblotting results (n = 6, one-way ANOVA) (data are represented by mean ± SD; ns represents no statistically significant difference, *p < 0.05, ** p < 0.01, *** p < 0.005).
FIG. 10f illustrates quantitative analysis of active AEP/β-actin immunoblotting results (n = 6, one-way ANOVA) (data are represented by mean ± SD; ns represents no statistically significant difference, *p < 0.05, ** p < 0.01, *** p < 0.005).
FIG. 10g illustrates quantitative analysis of APP N585/APP N immunoblotting results (n = 6, one-way ANOVA) (data are represented by mean ± SD; ns represents no statistically significant difference, *p < 0.05, ** p < 0.01, *** p < 0.005).
FIG. 10h illustrates quantitative analysis of Tau N368/Tau 5 immunoblotting results (n = 6, one-way ANOVA) (data are represented by mean ± SD; ns represents no statistically significant difference, *p < 0.05, ** p < 0.01, *** p < 0.005).
FIG. 10i illustrates quantitative analysis of AT8/Tau 5 immunoblotting results (n = 6, one-way ANOVA) (data are represented by mean ± SD; ns represents no statistically significant difference, *p < 0.05, ** p < 0.01, *** p < 0.005).
FIG. 11a illustrates immunofluorescent (IF) staining of p-TrkB and microtubule-associated protein 2 (MAP2) in brain tissues of 3xTg mice, with a scale bar = 50 µm.
FIG. 11b illustrates IF staining of p-AEP and MAP2 in brain tissues of 3xTg mice, with a scale bar = 50 µm.
FIG. 11c illustrates IF staining of AEP and APP C586 in brain sections of 3xTg mice, with a scale bar = 50 µm.
FIG. 11d illustrates IF staining of AT8 and Tau N368 in brain sections of 3xTg mice, with a scale bar = 50 µm.
FIG. 11e illustrates quantification of p-TrkB intensities in the hippocampus (n = 6, one-way ANOVA) (data are represented by mean ± SD; ns represents no statistically significant difference, *p < 0.05, ** p < 0.01, *** p < 0.001).
FIG. 11f illustrates quantification of p-AEP intensities in the hippocampus (n = 6, one-way ANOVA) (data are represented by mean ± SD; ns represents no statistically significant difference, *p < 0.05, ** p < 0.01, *** p < 0.001).
FIG. 11g illustrates quantification of AEP intensities in the hippocampus (n = 6, one-way ANOVA) (data are represented by mean ± SD; ns represents no statistically significant difference, *p < 0.05, ** p < 0.01, *** p < 0.001).
FIG. 11h illustrates quantification of APP C586 intensities in the hippocampus (n = 6, one-way ANOVA) (data are represented by mean ± SD; ns represents no statistically significant difference, *p < 0.05, ** p < 0.01, *** p < 0.001).
FIG. 11i illustrates quantification of Tau N368 intensities in the hippocampus (n = 6, one-way ANOVA) (data are represented by mean ± SD; ns represents no statistically significant difference, *p < 0.05, ** p < 0.01, *** p < 0.001).
FIG. 11j illustrates quantification of AT8 intensities in the hippocampus (n = 6, one-way ANOVA) (data are represented by mean ± SD; ns represents no statistically significant difference, *p < 0.05, ** p < 0.01, *** p < 0.001).
FIG. 12a illustrates effects of *in vitro* digestion time on the particle size of #2-loaded nanoparticle formulations.
FIG. 12b illustrates effects of *in vitro* digestion time on the bioaccessibility of #2-loaded nanoparticle formulations
FIG. 12c is scanning electron microscopy (SEM) images of #2/zein, #2/zein/LF, and #2/zein/DLF, with a magnification of ×40,000 and a scale bar = 2 µm.
FIG. 12d illustrates a viability of Caco-2 cells incubated with different concentrations of #2 for 24 h, and measured by a Cell Counting Kit-8 (CCK-8) assay.
FIG. 12e illustrates a measured viability of Caco-2 cells incubated with #2, #2/zein, #2/zein/LF, and #2/zein/DLF for 24 h.
FIG. 12f illustrates transepithelial electrical resistance (TEER) values of Caco-2 cells measured in a 21-day culture period.
FIG. 12g illustrates Caco-2 permeability data (n = 3; data are represented by mean ± SD).
FIG. 13a illustrates plasma concentrations and PK parameters of #2 quantitatively analyzed by LC-MS/MS after IG administration of #2, #2-NP, and #2-LIP at 5 mg/kg, as well as IN administration of #2 and #2-LIP at 5 mg/kg (n = 3; data are represented by mean ± SD).
FIG. 13b illustrates brain concentrations and PK parameters of #2 quantitatively analyzed by LC-MS/MS after IG administration of #2, #2-NP, and #2-LIP at 5 mg/kg, as well as IN administration of #2 and #2-LIP at 5 mg/kg (n = 3; data are represented by mean ± SD).

### DETAILED DESCRIPTIONS OF THE EMBODIMENTS

To understand the present disclosure more clearly, the present disclosure will be further described by reference to the following examples and drawings. The examples are used for explaining the present disclosure only, rather than limiting the present disclosure in any way. In the examples, all raw reagents and materials are commercially available, experimental methods not specified with detailed conditions are well-known conventional methods and conditions in the art, or are performed according to the recommended conditions of instrument manufacturers.

According to the present disclosure, four different derivatives (compounds #1-4) of CF3CN are synthesized by reducing CN groups of CF3CN into alkylamines and amides, where branched-chain lengths of the compounds are extended to enhance the lipophilicity without compromising agonist properties, thereby facilitating penetration across the blood brain barrier (BBB) and increasing the B/P Ratio of the compounds. Moreover, the compounds #1-4 are validated *in vitro* to activate the TrkB, and the effective concentrations for 50% of maximal effect (EC50) of the compounds are calculated. The absorption, distribution, metabolism, excretion, and toxicity (ADMET) properties of the candidate compounds #2-4 are analyzed, and the compound #2 is selected as the optimal compound for subsequent studies based on comprehensive evaluation. Although the compound #2 exhibits an EC50 value comparable to CF3CN and has a high stability, the compound #2 demonstrates a high efflux ratio in Caco-2 cell experiments, which indicates a poor oral bioavailability. The compound #2 is delivered via a zein/LF nanoparticle (#2/zein/LF), thereby enhancing the oral bioavailability. The pharmacokinetic-pharmacodynamic (PK/PD) and therapeutic effects of #2/zein/LF are investigated in AD mouse models.

The present disclosure is described in detail in combination with examples.

### Example 1

### 1. Synthesis of four CF3CN derivatives #1-4

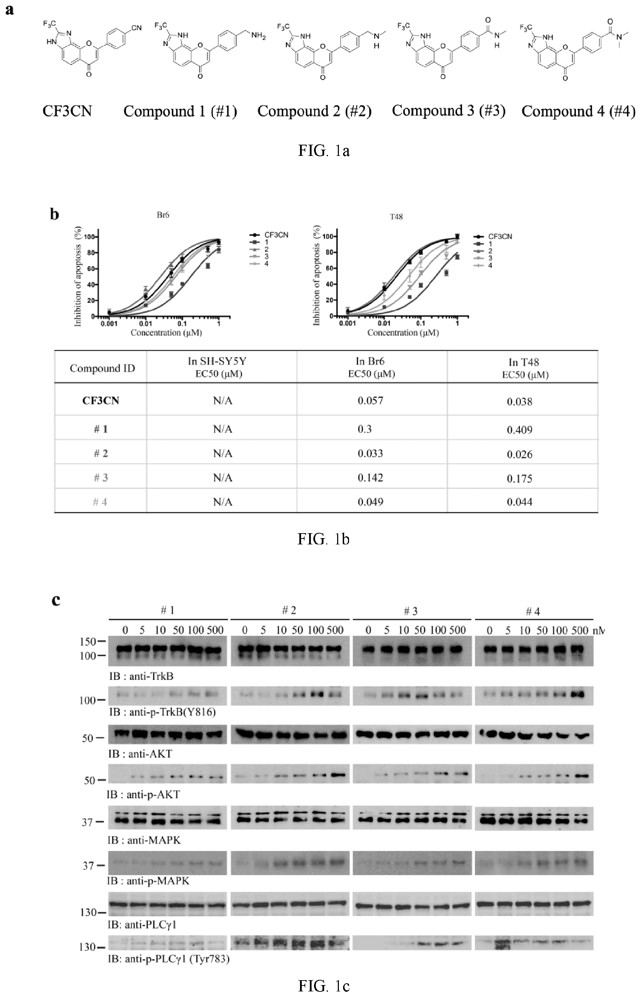

The structural formula of the compound CF3CN is illustrated in FIG. 1A. Four different derivatives (compounds #1-4) of CF3CN were synthesized by reducing CN groups of CF3CN to alkylamines and amides, with structural formulas of the four compounds illustrated in FIG. 1A. The detailed synthesis schemes for the four compounds are illustrated in FIG. 2. All chemical reagents and solvents utilized in this synthesis process were commercially purchased and used directly without further purification. ¹H NMR spectra were recorded on a Bruker Avance III 400 MHz spectrometer and a Bruker Fourier 300 MHz spectrometer, with tetramethylsilane (TMS) used as an internal standard. Liquid chromatography/mass spectrometry (LC/MS) analysis was performed on an Agilent LC/MSD 1200 series quadrupole mass spectrometer under the following conditions: column: ODS 2000 (150 × 4.6 mm, 5 µm), and ionization mode: ES(+) or ES(-); column temperature: 30°C; flow rate: 1.5 mL/min; and detection wavelengths: 214 nm and 254 nm. HPLC conditions: column: Welchrom C18, 150 × 20 mm; detection wavelength: 220 nm; mobile phase: A: MeCN containing 0.1% (v/v) trifluoroacetic acid (TFA), and B: water containing 0.1% (v/v) TFA; flow rate: 25 mL/min; injection volume: 2 mL; detection run time: 30 min; and column equilibration time: 5 min.

Preparation of R2: At 0°C, 79.2 mL of BH3/THF solution (1.0 M, 79.2 mmol) was added to 100 mL of THF solution containing R1 (10.5 g, 39.6 mmol), and the reaction mixture was stirred at room temperature overnight. Then, the mixture was quenched with MeOH (20 mL), concentrated, and purified by a silica gel column chromatography using petroleum ether containing 15% (v/v) ethyl acetate as an eluent to obtain R2 as a brown oil (10.4 g, 100% yield). A molecular weight of R2 was measured to be 252.3.

Preparation of R3c: R2 (10.0 g, 40.0 mmol) was dissolved in 150 mL of DCM solution, and NaHCO₃ (6.72 g, 80.0 mmol) and DMP (18.6 g, 44.0 mmol) were added to the solution under a nitrogen atmosphere at 0°C. The reaction mixture was stirred at room temperature for 2 h, and then the product was washed with saturated aqueous NaHCO₃ solution (3 × 50 mL) and brine (2 × 50 mL). The resulting organic portion was dried by anhydrous Na₂SO₄, filtered, concentrated, and purified by a silica gel column chromatography using petroleum ether containing 15% (v/v) ethyl acetate as an eluent to obtain R3c as a yellow oil (8.66 g, 84% yield). A molecular weight of R3c was measured to be 250.3.

Preparation of R5: R4 (90 g, 0.58 mol) was dissolved in a solution of concentrated H₂SO₄ (400 mL), and HNO₃ (41 g, 0.64 mmol) was added to the mixture under a nitrogen atmosphere at 0°C. The reaction mixture was stirred at 0°C for 3 h, then poured into ice water (2,000 mL) and extracted with ethyl acetate (3 × 1,000 mL). The combined organic layers were washed with brine (3 × 500 mL), dried by anhydrous Na₂SO₄, filtered, concentrated, and purified by means of a silica gel column chromatography using a petroleum ether solution containing 20% (v/v) ethyl acetate as an eluent to obtain R5 as a yellow oil (33 g, 28% yield). A molecular weight of R5 was measured to be 200.2, and a structure of R5 was further identified by ¹H-NMR: ¹H-NMR (400 MHz, CDCl₃), 13.29 (s, 1H), 7.92 (dd, J=6.0 Hz, 9.2 Hz, 1H), 6.81 (t, J=8.8 Hz, 1H), 2.67 (s, 3H).

Preparation of R6: R5 (6.5 g, 32.7 mmol) was dissolved in DMSO (150 mL), and 15 mL of NH₃ H₂O was added to the mixture under a nitrogen atmosphere at 20°C. The reaction mixture was stirred at 50°C for 15 h, 10 mL of water was added to the mixture, and the reaction mixture was filtered to obtain filtrate. The filtrate was concentrated and dried to obtain R6 as a yellow solid (6.4 g, 100% yield). A molecular weight of R5 was measured to be 196.0.

Preparation of R7: R6 (6.4 g, 32.8 mmol) was dissolved in an ACN solution (60 mL), and 20 mL of 1N HCl was added to the suspension under a nitrogen atmosphere at 20°C. The reaction mixture was stirred at 90°C for 4 h, and concentrated to obtain R7 as a yellow solid (5.8 g, 92.1% yield). A molecular weight of R7 was measured to be 197.3.

Preparation of R8a: R7 (1.0 g, 5.1 mmol) was added to a suspension of NaH (714 mg, 17.9 mmol) in anhydrous DMF (30 mL) under a nitrogen atmosphere at 0°C. The reaction mixture was stirred at 0°C for 1 h, and then R3a (1.67 g, 12.8 mmol) was added to the reaction mixture. The reaction mixture was further stirred at room temperature for 3 h. 100 mL of water was added to the reaction mixture, and the mixture was extracted with ethyl acetate (2 × 100 mL). Organic layers were combined, washed with brine (3 × 100 mL), dried by anhydrous Na₂SO₄, filtered, and concentrated. The residue was purified by a silica gel column chromatography using CH₂Cl₂ containing 10% (v/v) MeOH as an eluent to obtain R8a as a yellow solid (1.5 g, 91% yield). A molecular weight of R8a was measured to be 310.3.

Preparation of R9a: R8a (1.5 g, 4.85 mmol) was dissolved in a solution of DMSO (10 mL), and I₂ (185 mg, 0.72 mmol) was added to the solution. The mixture was stirred at 130°C for 2 h. Upon completion of the reaction, the reaction mixture was quenched with ice water (20 mL) and filtered. The upper filter cake was dried to obtain R9a as a yellow solid (1.35 g, 90% yield). A molecular weight of R9a was measured to be 307.9.

Preparation of R10a: R9a (1.35 g, 4.4 mmol) was dissolved in a solution of pyridine (14 mL), and TFAA (2.77 g, 13.2 mmol) was added to the solution. Under a nitrogen atmosphere at room temperature, the reaction mixture was stirred for 2 h, and quenched with water (20 mL). The precipitate was filtered, and the resulting filtrate was concentrated to obtain R10a as a yellow solid (1.6 g, 90% yield). A molecular weight of R10a was measured to be 404.3.

Preparation of R11a: R10a (1.6 g, 4.0 mmol) was dissolved in a mixed solution of MeOH/AcOH (20 mL/10 mL), iron powder (1.12 g, 20.0 mmol) was added to the solution, and the mixture was stirred at 50°C for 2 h. After cooling to room temperature, the reaction mixture was filtered, and the resulting filtrate was concentrated. The residue was purified by a silica gel column chromatography using petroleum ether containing 50% (v/v) ethyl acetate as an eluent to obtain R11a as a yellow solid (730 mg, 52% yield). A molecular weight of R11a was measured to be 356.3.

Preparation of compound #1: R11a (730 mg, 2.2 mmol) was dissolved in a solution of DMF (5 mL), and Ra-Ni (200 mg) was added to the solution. The mixture was stirred under hydrogen gas (1 atm) at 50°C for 12 h. After cooling to room temperature, the reaction mixture was filtered, and the resulting filtrate was concentrated. The resulting residue was purified by a C18 column chromatography (120 g C18 column, flow rate: 50 mL/min, and mobile phase: 1,800 mL of 5%-95% aqueous ACN solution) to obtain #1 as a yellow solid (140 mg, 20% yield). A molecular weight of the compound #1 was measured to be 360.0. A structure of the comopund was analyzed by ¹H-NMR (400 MHz, DMSO-d6): 8.30-8.22 (m, 5H), 8.05-8.03 (m, 1H), 7.78-7.70 (m, 3H), 7.23 (s, 1H), 4.18 (s, 2H).

Preparation of R8b: R7 (5.0 g, 25.5 mmol) was dissolved in anhydrous DMF (180 mL). Under a nitrogen atmosphere at 0°C, a suspension of NaOH (3.57 g, 89.3 mmol) was added. The reaction mixture was stirred at 0°C for 1 h, and then R3b (13.1 g, 63.7 mmol) was added to the reaction mixture. The reaction mixture was further stirred at room temperature for 3 h. 200 mL of water was added, and the mixture was extracted with ethyl acetate (2 × 200 mL). Organic layers were mixed, washed with brine (3 × 100 mL), dried by anhydrous Na₂SO₄, filtered, and concentrated. The residue was purified by a silica gel column chromatography using CH₂Cl₂ containing 10% (v/v) MeOH as an eluent to obtain R8b as a yellow solid (3.0 g, 26% yield). A molecular weight of R8b was measured to be 385.3.

Preparation of R9b: R8b (3.0 g, 8.0 mmol) was dissolved in a solution of DMSO (30 mL), and I₂ (300 mg, 1.1 mmol) was added to the solution. The reaction mixture was stirred at 130°C for 2 h, and quenched with ice water (20 mL). The upper filter cake was dried to obtain R9b as a yellow solid (3.0 g, 90% yield). A molecular weight of R9b was measured to be 383.3.

Preparation of R10b: R9b (2.9 g, 7.6 mmol) was dissolved in a solution of pyridine (50 mL), TFAA (4.78 g, 22.8 mmol) was added to the solution, and the reaction was allowed at 0°C. The reaction mixture was stirred under a nitrogen atmosphere at room temperature for 2 h. The mixture was quenched with water (50 mL), the precipitate was filtered, and the filter cake was dried to obtain R10b as a yellow solid (3.46 g, 95% yield). A molecular weight of R10b was measured to be 479.3.

Preparation of R11b: R10b (3.36 g, 7.0 mmol) was dissolved in a mixed solution of MeOH/AcOH (50 mL/10 mL), and Fe (1.97 g, 35.0 mmol) was added to the solution. The solution was stirred at 50°C for 2 h. After cooling to room temperature, the reaction mixture was filtered, and the resulting filtrate was concentrated. The residue was purified by a silica gel column chromatography using petroleum ether containing 50% (v/v) ethyl acetate as an eluent to obtain R11b as a yellow solid (2.2 g, 72% yield). A molecular weight of R11b was measured to be 431.3.

Preparation of R12: R11b (2.1 g, 4.88 mmol) was dissolved in a solution of DCM (25 mL), and TFA (8 mL) was added to the solution. The mixture was stirred at room temperature for 2 h, and concentrated to obtained R12 as a yellow solid (1.9 g, 100% yield). A molecular weight of R12 was measured to be 375.3.

Preparation of compound #3: R12 (240 mg, 0.64 mmol) and CH₃NH₂ (2.0 M, 0.35 mL, 0.71 mmol) were dissolved in DMF (7 mL), DIEA (165 mg, 1.28 mmol) and HATU (293 mg, 0.77 mmol) were respectively added at 0°C, and the mixture was heated under a nitrogen atmosphere. The reaction mixture was stirred at room temperature for 2 h, and concentrated and purified by a C18 column chromatography (120 g C18 column, flow rate: 50 mL/min, and mobile phase: 1,800 mL of 5%-95% ACN aqueous solution) to obtain a compound #3 as a yellow solid (134 mg, 56% yield). A molecular weight of the compound #3 was measured to be 388.3. A structure of the compound #3 was identified by ¹H-NMR: ¹H-NMR (400 MHz, DMSO-d6), 8.66 (d, J=4.8 Hz, 1H), 8.30 (brs, 2H), 8.07-8.01 (m, 3H), 7.78-7.76 (m, 1H), 7.26 (s, 1H), 2.84 (d, J=4.4 Hz, 3H).

Preparation of compound #4: R12 (240 mg, 0.64 mmol) and dimethylamine hydrochloride (58 mg, 0.71 mmol) were dissolved in a solution of DMF (7 mL), and DIEA (165 mg, 1.28 mmol) and HATU (293 mg, 0.77 mmol) were added sequentially under a nitrogen atmosphere at 0°C. The reaction mixture was stirred at room temperature for 2 h, and concentrated and purified by a C18 column chromatography (120 g C18 column, flow rate: 50 mL/min, and mobile phase: 1,800 mL of 5%-95% aqueous ACN solution) to obtain a compound #4 as a yellow solid (137 mg, 57% yield). A molecular weight of the compound #4 was measured to be 402.3, and a structure of the compound #4 was identified by ¹H-NMR: ¹H-NMR (400 MHz, DMSO-d6), 8.26 (brs, 2H), 8.04-8.01 (m, 1H), 7.78-7.75 (m, 1H), 7.65 (d, J=8.4 Hz, 2H), 7.23 (s, 1H), 3.03 (s, 3H), 2.95 (s, 3H).

Preparation of R8c: R7 (2.9 g, 14.8 mmol) was dissolved in a solution of anhydrous DMF (100 mL), and a suspension of NaH (2.1 g, 51.8 mmol) was added to the solution under a nitrogen atmosphere at 0°C. The reaction mixture was stirred at 0°C for 1 h, and R3c (8.1 g, 51.7 mmol) was added to the reaction mixture. The reaction mixture was further stirred at room temperature for 3 h. 200 mL of water was added, and the mixture was extracted with ethyl acetate (2 × 200 mL). Organic layers were mixed, washed with brine (3 × 100 mL), dried by anhydrous Na₂SO₄, filtered, and concentrated. The residue was purified by a silica gel column chromatography using CH₂Cl₂ containing 10% (v/v) MeOH as an eluent to obtain R8c as a yellow solid (3.9 g, 62% yield). A molecular weight of R8c was measured to be 428.3.

Preparation of R9c: R8c (2.7 g, 6.3 mmol) was dissolved in a solution of DMSO (60 mL), and I₂ (240 mg, 0.95 mmol) was added to the solution. The solution was stirred at 130°C for 2 h, and upon completion of the reaction, the reaction mixture was quenched with ice water (20 mL), and filtered. The filter cake was concentrated to obtain R9c as a yellow solid (600 mg, 18% yield). A molecular weight of R9c was measured to be 426.3.

Preparation of R10c: R9c (500 mg, 1.2 mmol) was dissolved in a solution of pyridine (10 mL), TFAA (740 mg, 3.6 mmol) was added to the solution, the reaction temperature was controlled at 0°C, and the reaction mixture was stirred under a nitrogen atmosphere at room temperature for 2 h. The mixture was quenched with water (50 mL), and the precipitate was filtered. The filter cake was dried to obtain R10c as a yellow solid (735 mg, 95% yield). A molecular weight of R10c was measured to be 522.3.

Preparation of R11c: R10c (680 mg, 1.3 mmol) was dissolved in a mixed solution of MeOH/AcOH (10 mL/2 mL), and iron powder (365 mg, 6.5 mmol) was added to the solution. The solution was stirred at 50°C for 2 h. After cooling to room temperature, the reaction mixture was filtered, and the resulting filtrate was concentrated. The residue was purified by a silica gel column chromatography using petroleum ether containing 50% (v/v) ethyl acetate as an eluent to obtain R11c as a yellow solid (560 mg, 92% yield). A molecular weight of R11c was measured to be 474.3.

Preparation of compound #2: R11c (360 mg, 0.76 mmol) was dissolved in a solution of DCM (9 mL), and TFA (3 mL) was added to the solution. The mixture was stirred at room temperature for 2 h, and concentrated and purified by a C18 column chromatography (120 g C18 column, flow rate: 50 mL/min, and mobile phase: 1,800 mL of 5%-95% aqueous ACN solution) to obtain a compound #2 as a yellow solid (130 g, 44% yield). A molecular weight of the compound #2 was measured to be 374.3, and a structure of the compound #2 was identified by ¹H-NMR: ¹H-NMR (400 MHz, DMSO-d6), 9.09 (brs, 2H), 8.29-8-27 (m, 2H), 8.02 (d, J=8.4 Hz, 1H), 7.78-7.72 (m, 3H), 7.23 (s, 1H), 4.27 (s, 2H), 2.63 (s, 3H).

### 2. Reduction derivatives #1-4 of CF3CN cyanides and EC50 values of activated TrkB receptors thereof

The pro-survival effects of four derivatives were quantitatively analyzed in human SH-SY5Y cells and T48 cells stably transfected with the TrkB. After induction of apoptosis by staurosporine (STS), all the four derivatives exhibited TrkB-dependent anti-apoptotic effects in both Br6 and T48 cells. The pro-survival EC50 values of compounds #2 and #4 were comparable to the pro-survival EC50 value of CF3CN (FIG. 1b). Immunoblotting results demonstrated that these compounds activated p-TrkB and downstream effectors p-Akt and p-MAPK in a dose-dependent manner. As PLCγ1 bound with a p-Y816 residue on the TrkB receptor and was subsequently tyrosine-phosphorylated by active TrkB, a tyrosine phosphorylation signal was also monitored in this example (FIG. 1c).

### 3. In vitro ADMET analysis

Due to the excessively high EC50 value of the compound #1, the applicant selected compounds #2-4 for *in vitro* Absorption, Distribution, Metabolism, Excretion, Toxicity (ADMET) analysis, and compared analysis results to CF3CN. As illustrated in Table 1, most indexes were similar between the compounds; and the compound #2 exhibited a superior stability in hepatic microsomes and bound with minimal plasma proteins, indicating that the compound #2 was an optimal derivative.

**Table 1 Comparison of in vitro ADMET between CF3CN and compounds #2-4**

| | | CF3CN | #2 | #3 | #4 |
|---|---|---|---|---|---|
| Solubility (pH7.4: PBS) | | >200 µM | 50 µM | >100 µM | >100 µM |
| Stability-Microsomes (Species) | T_{1/2} (min) | > 180(human); | 598(human); | 1350(human); | -187(human); |
| | | >180(mouse) | 133(mouse) | -268 (mouse) | -939 (mouse) |
| | CLᵢₙₜ (uL/min/mg) | <12.8(human); | 2.32(human); | 1.03(human); | -7.43(human); |
| | | <12.8(mouse) | 10.5(mouse) | -5.17(mouse) | -1.48(mouse) |
| Stability-Hepatocytes (Species) | T_{1/2} (min) | >480 (mouse); | >480 (mouse); | >480 (mouse); | >480 (mouse); |
| | | >480 (human) | >480 (human) | >480 (human) | >480 (human) |
| | CLᵢₙₜ (mL/min/10⁶ Cells) | <2.9(human); | <3(human); | <3(human); | <3(human); |
| | | <2.9(mouse) | <3(mouse) | <3(mouse) | <3(mouse) |
| Stability-Plasma (species) | T_{1/2} (min) | >480 (mouse); | >480 (mouse); | >480 (mouse); | >480 (mouse); |
| | | >480 (human) | >480 (human) | >480 (human) | >480 (human) |
| Plasma Protein Binding (species) | Fu, plasma (%) | 0.101% (human); | 4.6% (human); | 0.2% (human); | 0.4% (human); |
| | | 3.55% (mouse) | 1.2% (mouse) | 0.5% (mouse) | 0.9% (mouse) |
| Permeability -PAMPA-BBB | Pe (10⁻⁶ cm/s) | 3.09 | 0.135 | 0.815 | 1.27 |
| Permeability -Caco-2 | Papp (a-b, b-a, 10⁻⁶ cm/s) | 13.0(a-b); | 0.0175 (a-b); | ND (a-b); | ND(a-b); |
| | | 42.6 (b-a) | 13.9 (b-a) | 28.7 (b-a) | 45.9 (b-a) |
| | Efflux Ratio | 3.6 | 193 | NC | NC |
| hERG-(method) | IC₅₀ (µM) | >25 µM | >10 µM | >10 µM | >10 µM |
| Cₘₐₓ (ng/mL) | | 11267.967±2675.791 | 90.809±18.769 | - | - |
| Tₘₐₓ (h) | | 0.833±0.289 | 0.36±0.242 | - | - |
| t_{1/2} (h) | | 2.994±1.539 | 4.092±2.646 | - | - |
| Oral bioavailability (%) | | 136 | 6.99 | - | - |

### Example 2

### 1. Preparation and characterization of compound #2/zein/LF nanoparticle formulations

### (1) Determination of weight ratio of the compound #2 to zein

Compound #2/zein nanoparticle formulations with different weight ratios of the compound #2 to zein were prepared by an anti-solvent co-precipitation method (ASCP). Specifically, the compound #2 and zein were dissolved in an 80 vol% aqueous ethanol solution to obtain a compound #2/zein aqueous ethanol solution, and the compound #2/zein aqueous ethanol solution was added to deionized water under stirring. After solvent evaporation, the compound #2/zein nanoparticle formulations were obtained.

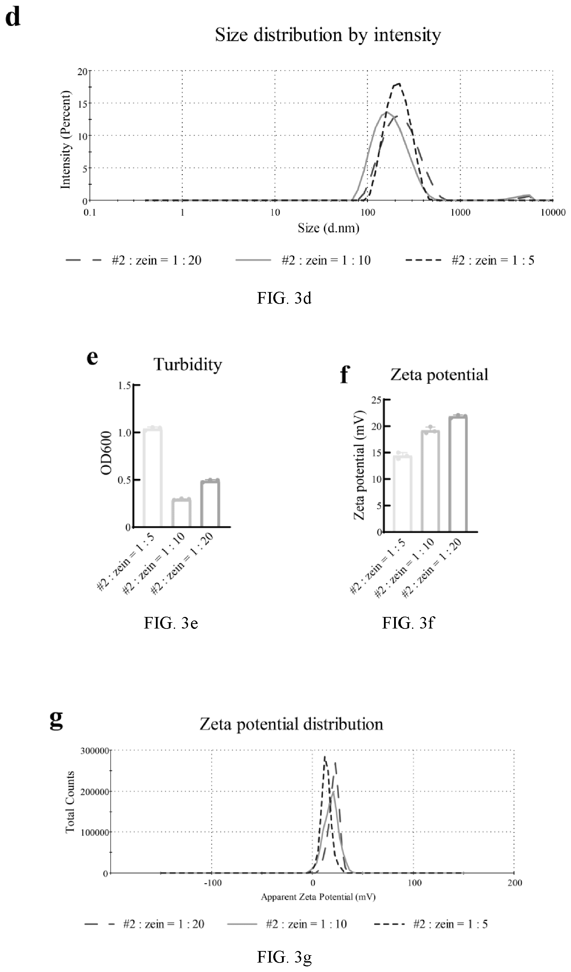

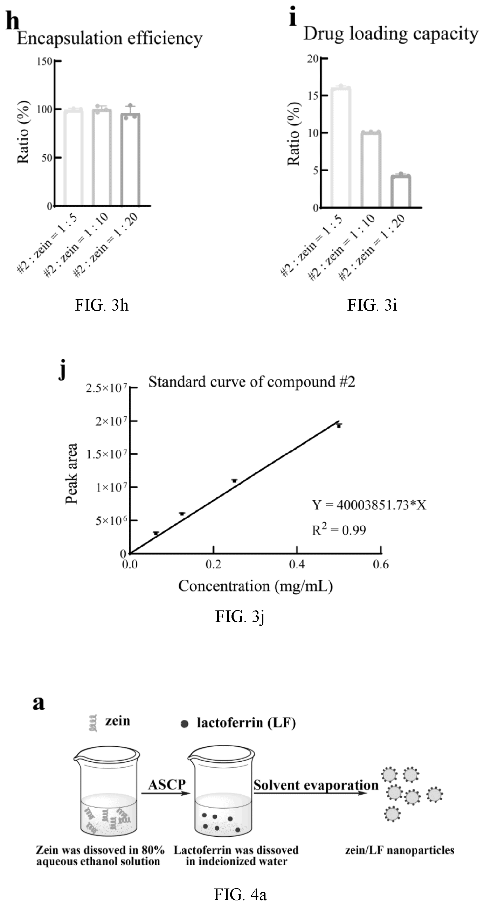

The compound #2/zein nanoparticle formulations were characterized for the appearance, particle size, zeta potential, polydispersity index (PDI), and turbidity of the nanoparticle formulations; and the encapsulation efficiency (EE) and drug loading capacity (LC) of the nanoparticle formulations for the compound #2. The results are shown in FIG. 3.

**Table 2 Physical properties of nanoparticle formulations prepared from the compound #2 and zein at different weight ratios**

| Formulation | Size (nm) | PDI | Zeta Potential (mV) | Turbidity | EE (%) | LC (%) |
|---|---|---|---|---|---|---|
| #2 : zein = 1 : 5 | 212.333±4.954 | 0.099±0.012 | 14.4±0.6 | 1.04±0.018 | 99.038± 1.939 | 16.07±0.258 |
| #2 : zein = 1 : 10 | 166.633±2.309 | 0.184±0.012 | 19.2±0.624 | 0.294±0.007 | 99.758±3.387 | 10.082±0.038 |
| #2 : zein = 1 : 20 | 197.367±3.523 | 0.187±0.014 | 21.833±0.306 | 0.489±0.013 | 95.681±7.150 | 4.301±0.206 |

According to the above results, an optimal weight ratio of the compound #2 to the zein was determined to be 1:10.

### (2) Determination of weight ratio of zein to LF

LF, an iron-binding glycoprotein, functions as a stabilizing agent for nanoparticles. Zein/LF nanoparticle formulations with different weight ratios of the zein to the LF were prepared by an ASCP. Specifically, the zein was dissolved in an 80 vol% aqueous ethanol solution to obtain a zein aqueous ethanol solution, and the zein aqueous ethanol solution was added to deionized water under stirring. After solvent evaporation, the zein/LF nanoparticle formulations were obtained.

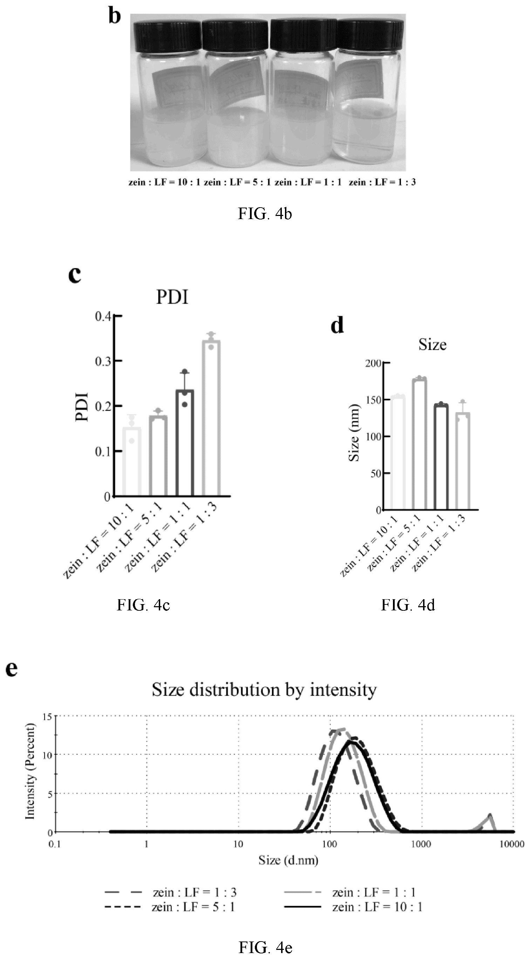

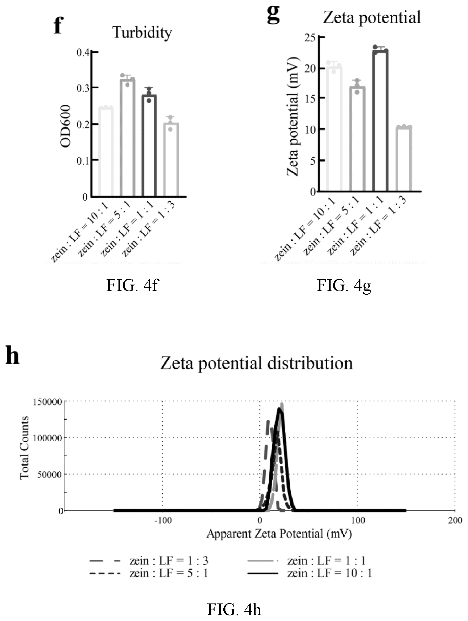

The zein/LF nanoparticle formulations were characterized for the appearance, particle size, zeta potential, PDI, and turbidity. The results are shown in FIG. 4.

**Table 3 Physical properties of nanoparticle formulations prepared from the zein and LF at different weight ratios**

| Formulation | Size (nm) | PDI | Zeta potential (mV ) | Turbidity |
|---|---|---|---|---|
| zein: LF = 10 : 1 | 154.4 33± 1.457 | 0.153 ± 0.027 | 20.233 ± 0.764 | 0.246 ± 0.001 |
| zein : LF = 5 : 1 | 178.233±1.976 | 0.178 ± 0.01 | 17± 0.954 | 0.324 ± 0.014 |
| zein : LF = 1 : 1 | 142.933 ± 1.617 | 0.236 ± 0.037 | 22.9 ± 0.458 | 0.283 ± 0.01 7 |
| zein : LF = 1 : 3 | 132.6 ± 13.116 | 0.346 ± 0.015 | 10.433 ± 0.058 | 0.203 ± 0.01 7 |

According to the above results, an optimal weight ratio of the zein to the LF was determined to be 1: 1.

### (3) Characterization of compound #2/zein, compound #2/zein/LF, and compound #2/zein/DLF nanoparticle formulations

The compound #2/zein nanoparticle formulation was prepared by an ASCP: the compound #2 and zein at a weight ratio of 1:10 were dissolved in an 80 vol% aqueous ethanol solution to obtain a compound #2/zein aqueous ethanol solution, and the compound #2/zein aqueous ethanol solution was added to deionized water under stirring. After solvent evaporation, the compound #2/zein nanoparticle formulation was obtained.

The compound #2/zein/LF nanoparticle formulation was prepared by an ASCP: the compound #2, zein, and LF at a weight ratio of 1:10:10 were dissolved in an 80% (v/v) aqueous ethanol solution to obtain a compound #2/zein/LF aqueous ethanol solution, and the compound #2/zein/LF aqueous ethanol solution was added to deionized water under stirring. After solvent evaporation, the compound #2/zein/LF nanoparticle formulation was obtained.

The compound #2/zein/DLF nanoparticle formulation was prepared by an ASCP: within a low pH range, the compound #2/zein/LF nanoparticle formulation exhibited a relatively poor salt tolerance. To stabilize the compound #2/zein/LF nanoparticle formulation, the LF was glycosylated via Maillard reaction, such that the compound #2/zein/DLF nanoparticle formulation was obtained. Specifically, the compound #2 and zein at a weight ratio of 1:10 were dissolved in an 80 vol% aqueous ethanol solution to obtain a compound #2/zein aqueous ethanol solution; the glycosylated LF was dissolved in deionized water to obtain a glycosylated LF aqueous solution, and the compound #2/zein aqueous ethanol solution was added to the glycosylated LF aqueous solution under stirring, with a weight ratio of the compound #2 to the zein to the glycosylated LF being 1:10:10. After solvent evaporation, the compound #2/zein/DLF nanoparticle formulation was obtained.

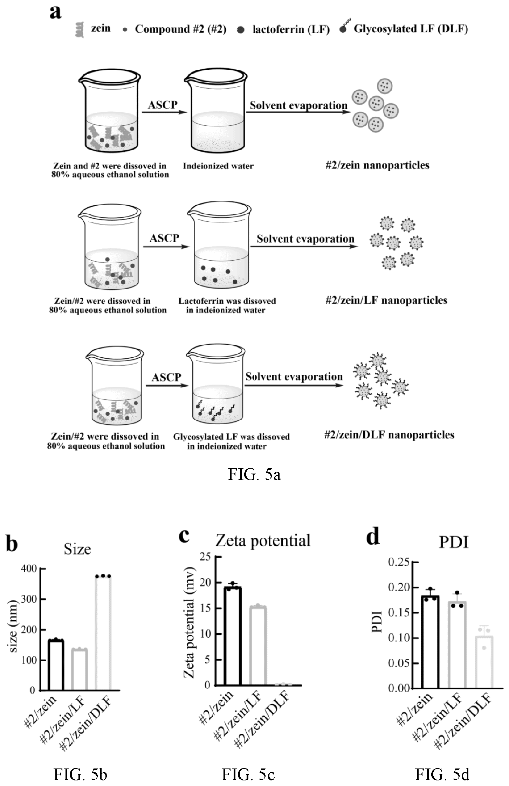

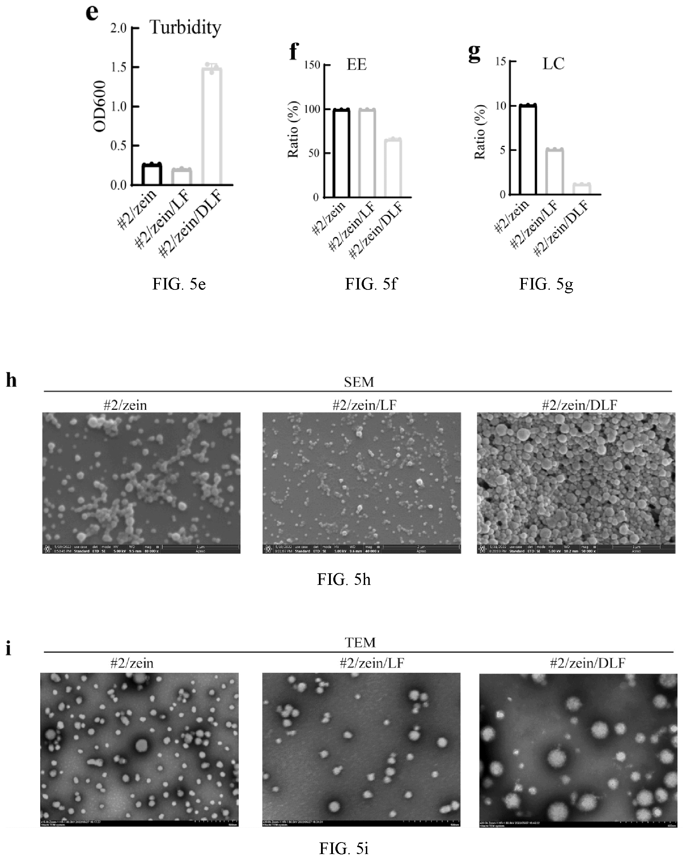

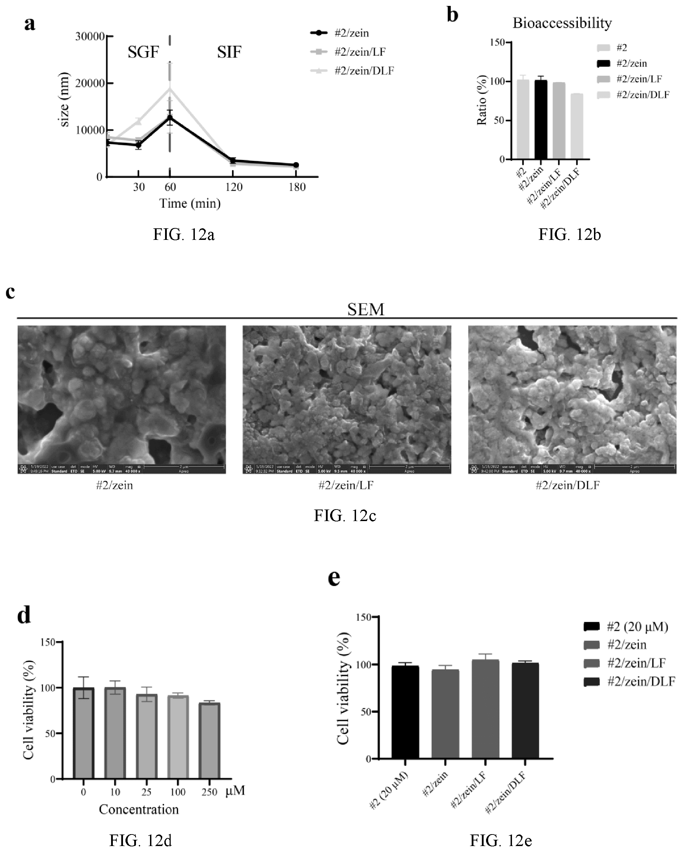

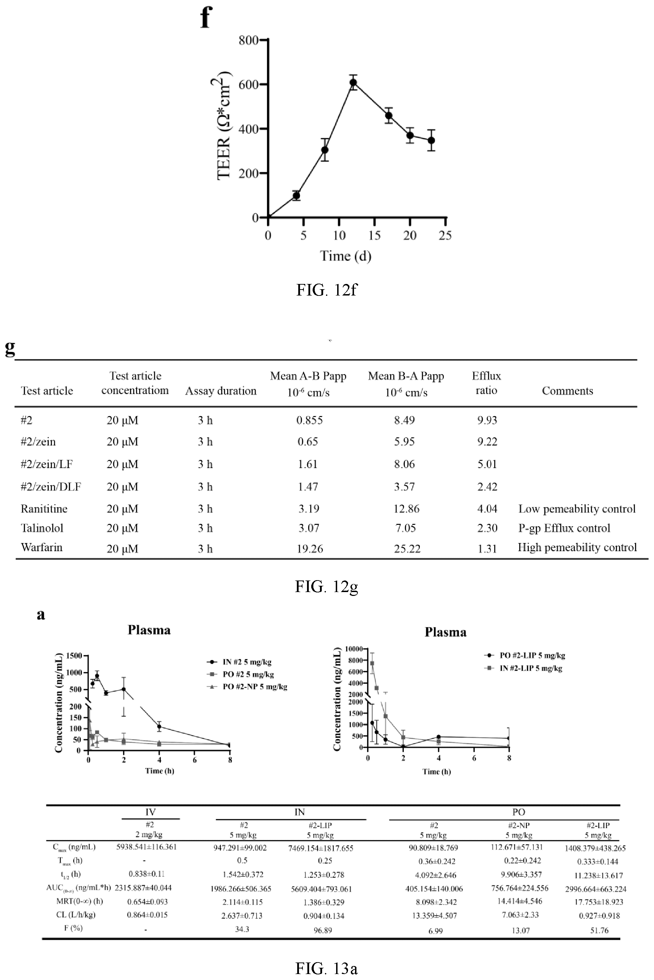

The #2/zein, #2/zein/LF and #2/zein/DLF nanoparticle formulations were characterized for the particle size, zeta potential, PDI, and turbidity of the nanoparticle formulations; and the EE and LC of the nanoparticle formulations for #2. The results demonstrated that the EE and LC of the #2/zein/DLF nanoparticle formulation were significantly reduced compared to the EE and LC of the #2/zein/LF nanoparticle formulation (FIGS. 5f & g). SEM and TEM results revealed that the particles were uniformly encapsulated into a spherical shape (FIGS. 5h & i). FIG. 12 illustrates bioaccessibility and Caco-2 uptake assays of #2-loaded nanoparticle formulations.

**Table 4 Physical properties of nanoparticle formulations**

| Formulation | Size (nm) | PDI | Zeta potential (mV) | Turbidity | EE (%) | LC (%) |
|---|---|---|---|---|---|---|
| #2/zein | 166.633±2.309 | 0.184±0.012 | 19.2±0.624 | 0.261±0.006 | 99.682±0.379 | 10.082±0.038 |
| #2/zein/LF | 136.733± 1.185 | 0.173±0.015 | 15.333±0.231 | 0.2±0.01 | 99.716±0.272 | 5.086±0.014 |
| #2/zein/DLF | 375.8± 1.153 | 0.104±0.02 | 0.19±0.094 | 1.491±0.053 | 65.605±1.295 | 1.175±0.023 |

The results demonstrated that the #2/zein/LF nanoparticle formulation exhibited an average particle size of approximately 136.7 nm, and was dispersed in a solution with a low turbidity (<0.22), a low PDI (<0.20), and a high EE (>99%). Based on analysis on the physicochemical properties of the three nanoparticle formulations, the #2/zein/LF nanoparticle formulation demonstrated optimal performance in most indexes and was therefore selected for further *in vivo* studies.

### 2. In vivo PK studies of compound #2 and nanoparticle formulations thereof (compound #2/zein/LF, designated as #2-NP)

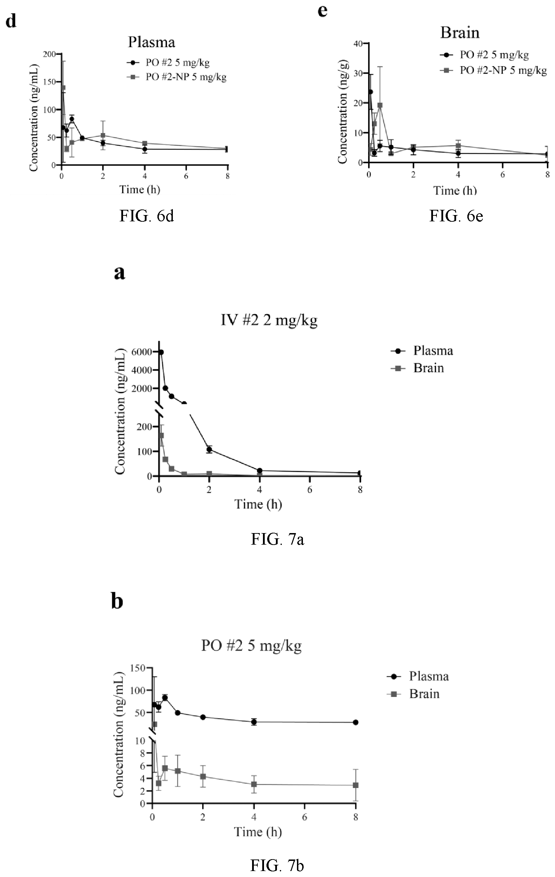

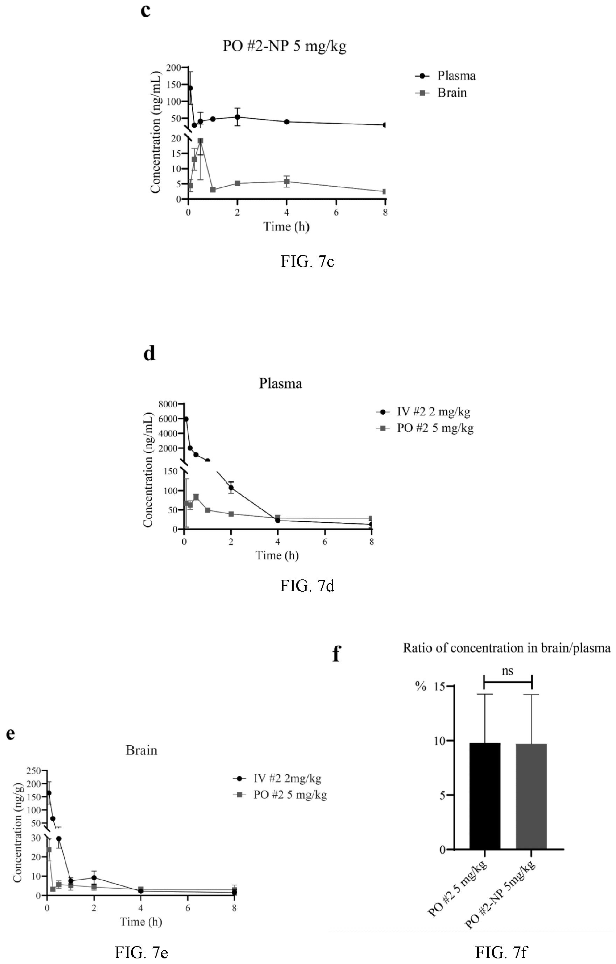

To investigate the *in vivo* PK profile of #2-NP, lyophilized #2-NP powder was redissolved. Although the redissolved nanoparticle exhibited a transparent appearance, both the EE and LC demonstrated slight reductions (FIG. 6A). For quantitative comparison of the effects of the nanoparticle system in delivering #2 to the blood circulation system and brain tissues, PK studies were performed in 2-month-old ICR mice. The results demonstrated that following PO administration of #2 at 5 mg/kg, a plasma half-life (t_{1/2}) was approximately 4.09 h, whereas the half-life was 0.84 h (2 mg/kg) following IV administration. The areas under curves (AUC) following IV administration and PO administration were 2315.89 ng/ml*h and 405.15 ng/ml*h, respectively. Notably, at identical doses of 5 mg/kg, the plasma t_{1/2} of #2-NP is 9.91 h, increasing by more than one-fold compared to the t_{1/2} (4.09 h) of #2. Comparative analysis of the nanoparticle-encapsulated compound administration and unencapsulated compound administration at identical doses revealed that the clearance (L/h/kg) decreased to 7.06 ng/ml*h from 13.36 ng/ml*h (unencapsulated), the AUC increased to 756.76 ng/ml*h from 405.15 ng/ml*h, and the maximum plasma concentration (Cₘₐₓ) rose to 112.67 ng/nm from 90.81 ng/nm. More importantly, after nanoparticle encapsulation, the oral bioavailability of the compound #2 was improved to 13.07% from 6.99% (unencapsulated) (FIG. 6b). Regarding the brain drug concentration, the Cₘₐₓ levels of #2 were 164.38 ng/g (IV administration) and 18.47 ng/g (PO administration), and the AUCs were 84.26 ng/ml*h and 35.75 ng/ml*h, respectively. The brain t_{1/2} levels were 1.41 h and 3.49 h, respectively. In contrast, PO administration of #2-NP at 5 mg/kg resulted in a brain AUC of 74.64, increasing by more than one-fold compared to PO administration of #2 (an AUC of 35.75) at identical doses. The t_{1/2} and Tₘₐₓ values of #2 and #2-NP were comparable (FIG. 6c). The *in vivo* plasma and brain PK profiles at a 5 mg/kg dose are depicted in FIGS. 6d & e. Detailed plasma and brain distribution analysis of #2 PK is shown in FIG. 7. Although the #2/zein/LF nanoparticle formulation improved the oral bioavailability of #2 and enhanced the brain exposure thereof, this nanoparticle delivery system did not significantly increase the B/P Ratio of #2 compared to #2 alone (FIG. 7F). However, the B/P Ratio of #2 reached approximately 10%, which demonstrated a marked improvement compared to CF3CN having a B/P Ratio of approximately 1%.

### 3. In vivo PK/PD studies of #2-NP

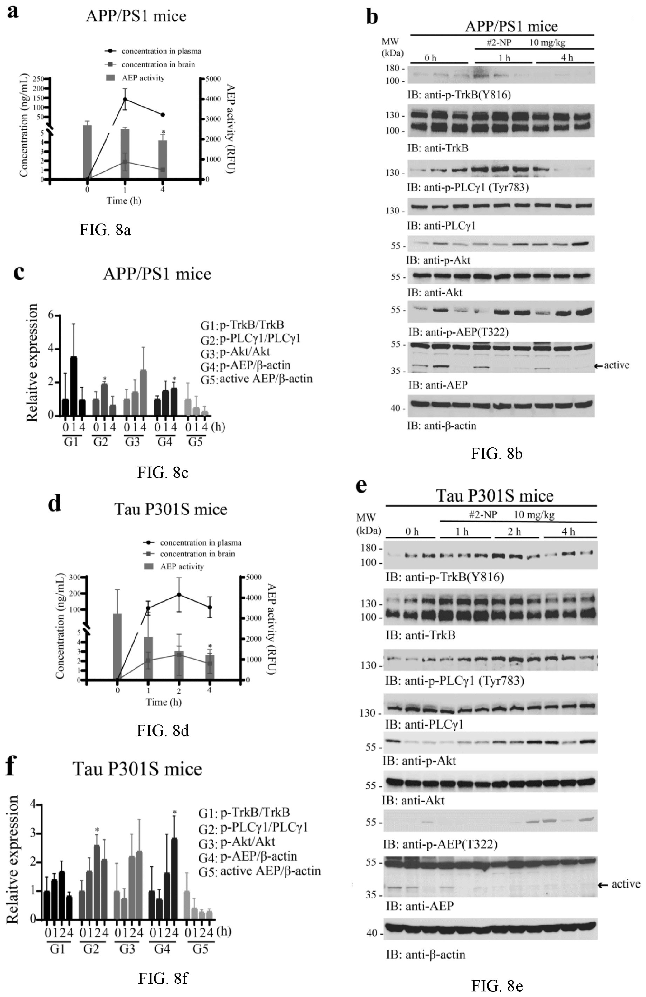

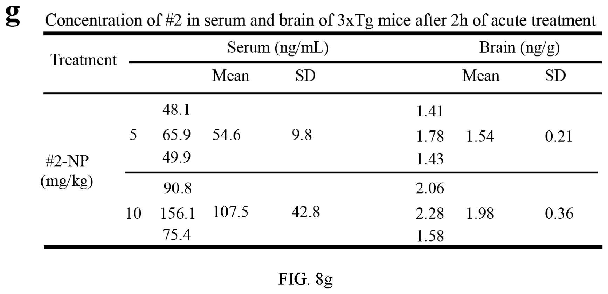

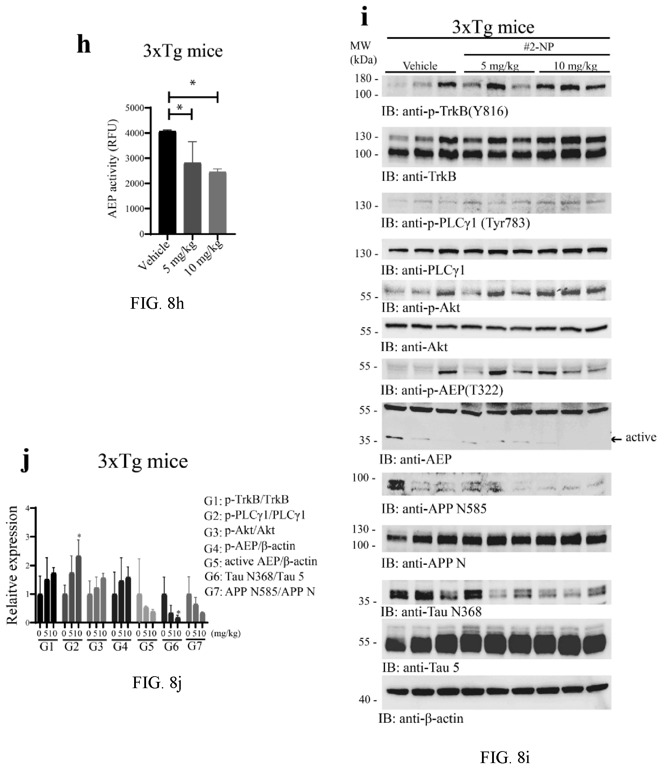

To evaluate whether #2-NP binds with a target TrkB receptor in the brain and triggers downstream inhibitory phosphorylation of AEP, APP/PS1 AD model mice were used, and analysis was performed on the AEP activities and the TrkB signaling pathways in brain tissue lysates following IG administration. The results demonstrated that quantitative LC/MS analysis was performed on a brain concentration of #2, and the AEP activity in the brain was correlated with an active AEP band level from analysis results, and exhibited an inverse relationship with the progressively increasing brain concentration of #2 (FIG. 8a). The immunoblotting results indicated the p-TrkB Y816 expression in the brain was enhanced at 1 h after PO administration of #2-NP, while a signal intensity decreased at 4 h. The downstream effector p-PLCγ1 Y783 exhibited similar PK profiles. Notably, p-Akt activation lasted 4 h, with AEP T322 being time-dependently phosphorylated by p-Akt. Akt was inversely coupled to AEP fragments, thereby triggering enzymatic activation of AEP (FIG. 8b). Similarly, quantitative analysis of immunoblotting (FIG. 8c) yielded results in full consistency with FIG. 8b. Similar PK analysis was conducted in Tau P301S mice and obtained similar observations: as the level of #2 in the brain increased, TrkB was subsequently activated, accompanied by an elevated p-AEP signal intensity and a reduced enzymatic activity (FIGS. 8d-f). To further investigate PK/PD relationships, #2-NP at 5 or 10 mg/kg were orally administered to 3xTg mice and the brain concentrations of #2 were analyzed after 2 h. Dose-dependent inhibition of the AEP activity by #2 was observed (FIGS. 8g & h). The immunoblotting results demonstrated that a p-TrkB signal intensity progressively increased with gradually elevated doses of #2-NP, accompanied by corresponding enhancement in the downstream effector p-PLCγ1 Y783. Consequently, the p-AEP T322 signal was consistent with upstream p-Akt activity, and was associated with reduced AEP cleavage. As predicted, fragments of AEP downstream substrates APP N585 and Tau N368 were steadily reduced with incremental #2-NP dosing (FIGS. 8i & j). Therefore, *in vivo* PK/PD studies support that #2 is targeted to TrkB receptors in the brain, and the AEP activity is inhibited via phosphorylation, thereby blocking cleavage of APP and Tau proteins.

### 4. Improvement of cognition, and reduction of Aβ deposition and brain atrophy in 3xTg mice by #2-NP

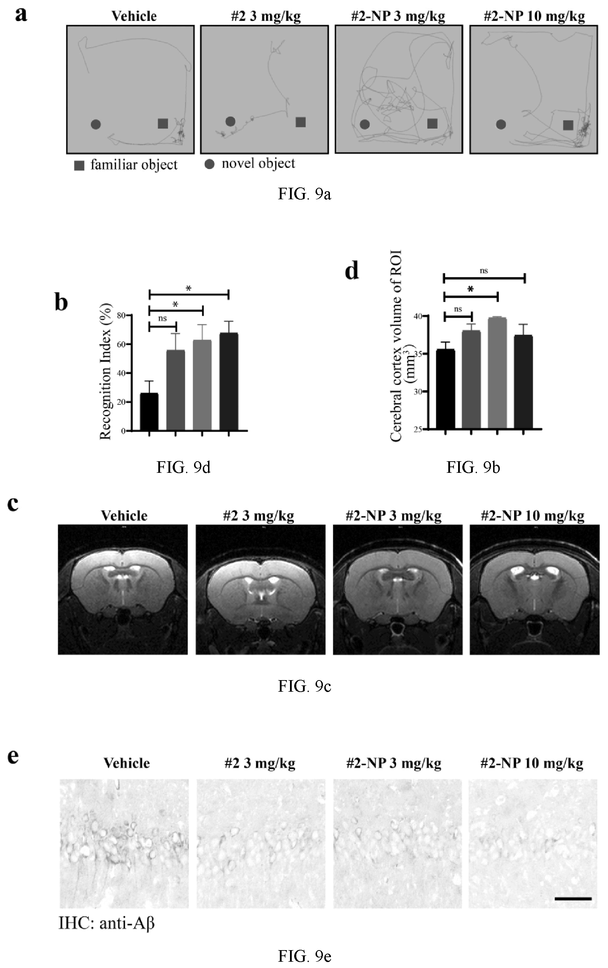

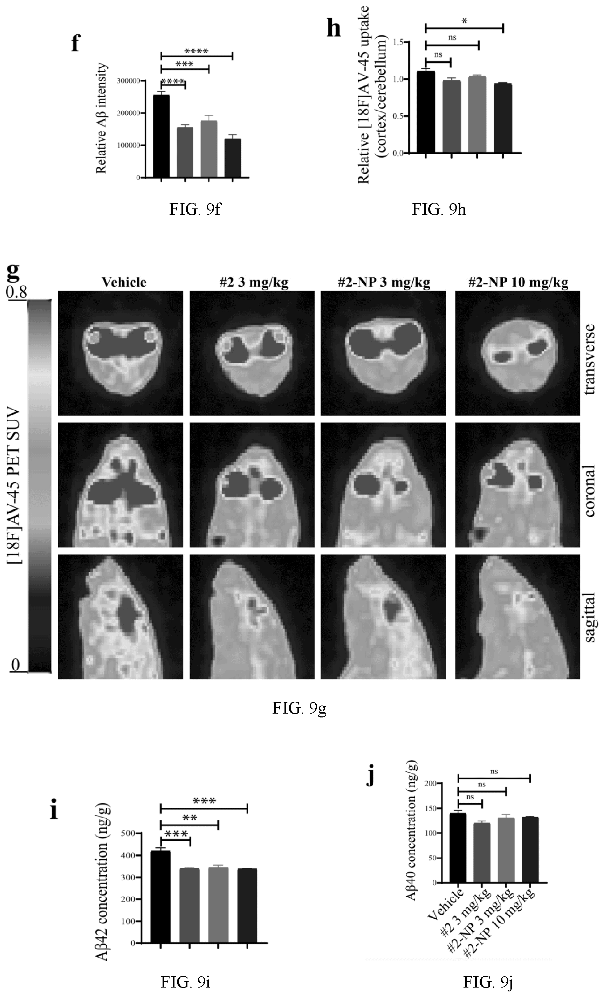

To investigate whether long-term chronic treatment with #2-NP alleviates cognitive deficits in 3xTg AD model mice, mice were administered via gavage with #2 at 3 mg/kg, #2-NP at 3 mg/kg, and #2-NP at 10 mg/kg. After 3 months of drug treatment, the NOR test demonstrated that #2 at 3 mg/kg failed to improve the cognitive function in 3xTg mice, whereas #2-NP significantly mitigated cognitive impairment. The recognition index was increased by #2-NP in a dose-dependent manner (FIGS. 9a and b). MRI scans revealed that #2-NP significantly increased the cerebral cortex volume compared to the Vehicle group, whereas #2 alone showed an elevation trend without statistical significance (FIGS. 9c & d). IHC staining of brain sections with an anti-Aβ antibody demonstrated that both #2 and #2-NP significantly reduced Aβ aggregation in the hippocampus, with #2-NP exhibiting a dose-dependent effect (FIGS. 9e & f). Additionally, quantitative analysis of Aβ PET signals was performed using the specific ligand [18F]AV-45, and the results revealed that #2-NP at 10 mg/kg significantly reduced the intensity of Aβ PET signals in 3xTg mice (FIGS. 9g & h). ELISA analysis demonstrated that both #2 and #2-NP significantly decreased the Aβ42 content in 3xTg mice, rather than the Aβ40 content (FIGS. 9i & j). Collectively, #2-NP decreases cerebral Aβ deposition, reduces brain atrophy, and ameliorates cognitive deficits in 3xTg mice.

### 5. Activation of TrkB signaling pathways and suppression of AEP activities in brains of 3xTg mice by #2-NP

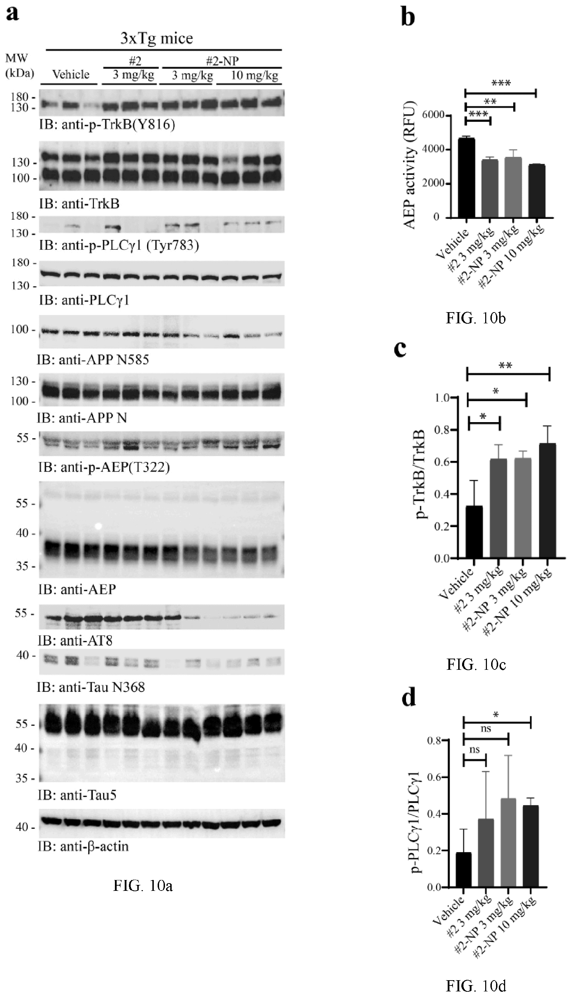

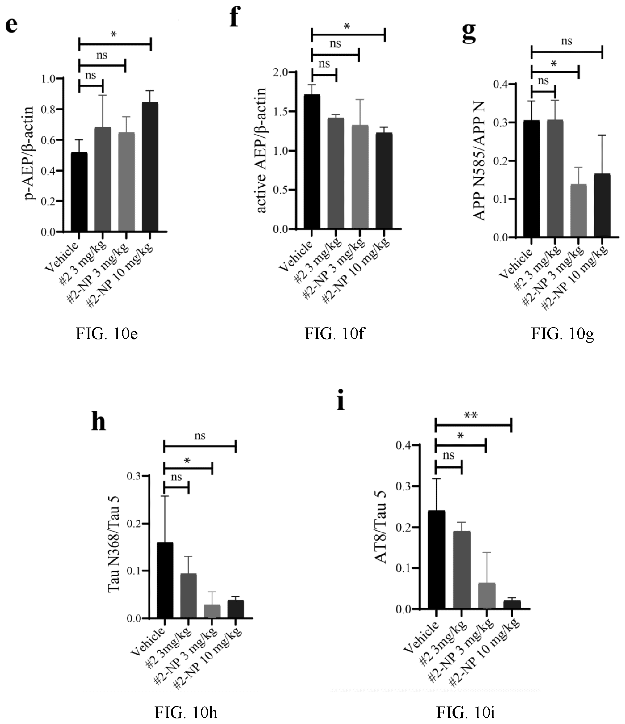

To investigate whether #2 biochemically activates TrkB signaling pathways and inhibits the AEP activities, immunoblotting analysis was performed on brain tissue lysates from 3xTg mice, and the results demonstrated that #2-NP dose-dependently activated the TrkB receptor, while p-PLCγ1 reflected upstream p-TrkB Y816 signaling pathways. Consequently, p-AEP T322 was correlated with the upstream TrkB signaling pathways which were inversely coupled with active AEP fragments. Cleavage of both APP N585 and Tau N368 was progressively reduced. Consistent with Tau N368 cleavage, p-Tau AT8 levels were progressively reduced (FIG. 10a). AEP enzymatic assays revealed that #2-NP exerted dose-dependent inhibition on the AEP activity. At identical doses, #2-NP exhibited a significantly higher biochemical activity than #2, which was consistent with the brain concentration of #2 increased by the nanoparticle system (FIGS. 10b-i).

### 6. Reduction of AD pathology in 3xTg mice by #2-NP

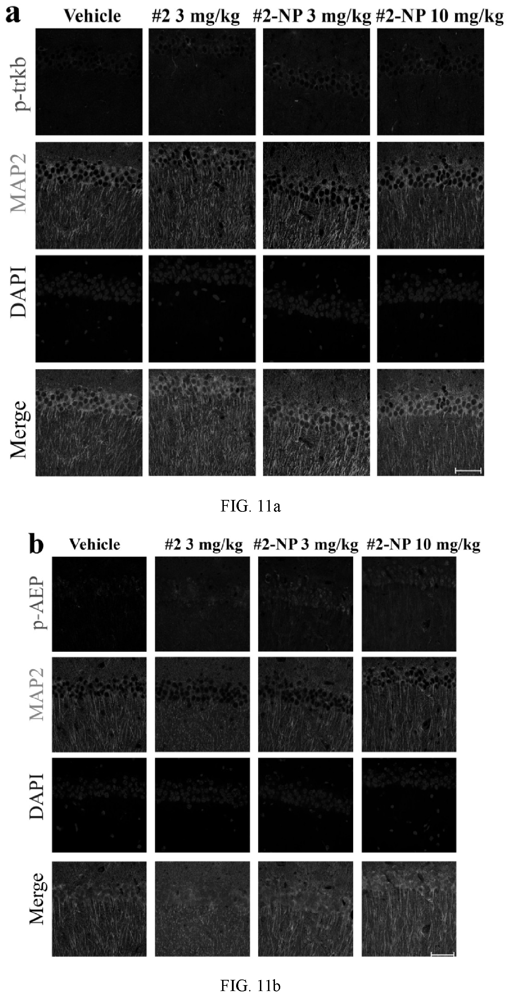

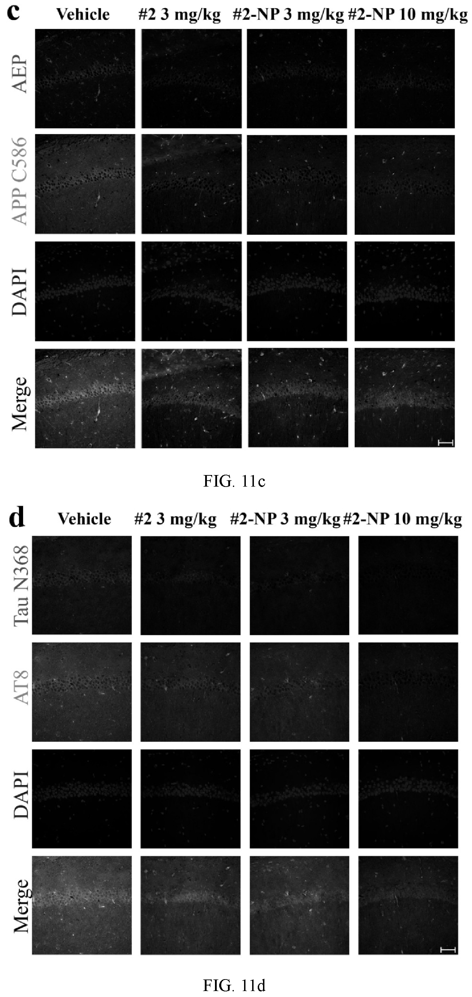

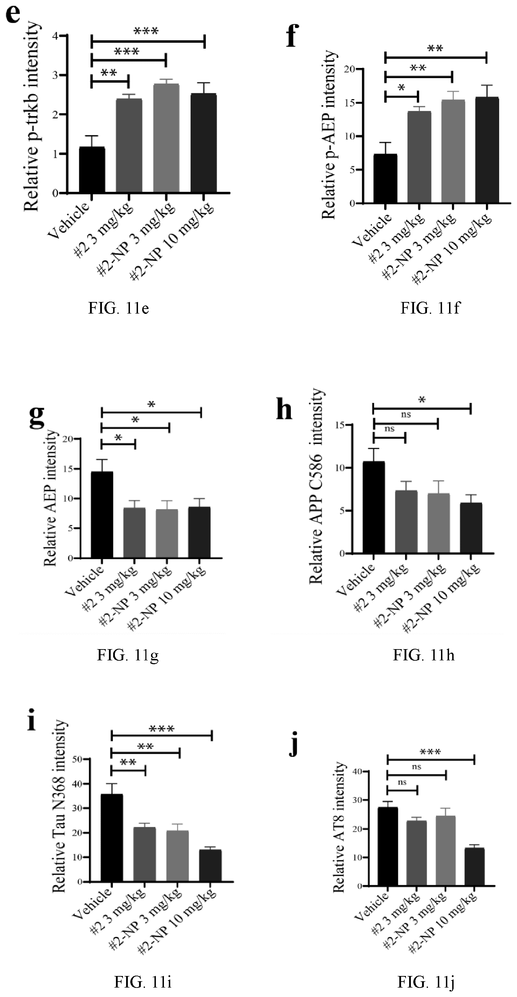

To further analyze the therapeutic effects of #2-NP against AD pathology, immunofluorescent (IF) staining of p-TrkB was performed on brain sections from drug-treated 3xTg mice. IF staining revealed significant activation of p-TrkB Y816 in the hippocampus by both #2 and #2-NP (FIGS. 7A & E). Consequently, p-AEP IF signals were closely correlated with the upstream p-TrkB activity (FIGS. 11b & f). Simultaneously, active AEP IF staining was inversely coupled with the p-AEP activity, thereby leading to a progressive decline in APP C585 levels. Consistent with these findings, Tau N368 signals and AT8 IF intensity were closely correlated with each other (FIGS. 7C & D). Quantitative analysis of the fluorescence intensity confirmed these observations (FIGS. 11g-j). Collectively, these results strongly support that #2-NP enhances the brain exposure of #2, increases TrkB activation, and inhibits the AEP activity in the brain, thereby ameliorating AD pathology and cognitive dysfunction in 3xTg mice.

### Example 3

### 1. Preparation and characterization of liposome

Preparation of compound #2-LIP: the compound #2 and lecithin (CAS No.: 8002-43-5) were dissolved in absolute ethyl alcohol to obtain a mixed solution, the mixed solution was added to deionized water under stirring, and the solvent was evaporated to obtain a crude liposome solution; the crude liposome solution was subjected to ice bath ultrasonication for 5-10 min; and chitosan was dissolved in deionized water to obtain an aqueous chitosan solution, and the liposome solution was added to the aqueous chitosan solution under stirring to obtain a compound #2-loaded liposome (# 2-LIP). Specifically, a weight ratio of the lecithin to the chitosan to the derivative was 20:2:1.

For the prepared liposome, physical characterization was first performed. The results (Table 5) demonstrated that #2-LIP exhibited a particle size of 298.77 nm, a PDI of 0.54, and a zeta potential of 23.97 mV, thereby indicating successful preparation of the liposome in this example. HPLC detection revealed an EE of 51.23% for the liposome, which was slightly lower than the EE of the nanoparticle.

**Table 5 Physical properties of liposome formulation**

| Formulation | Size (nm) | PDI | Zeta Potential (mV) | EE (%) |
|---|---|---|---|---|
| #2-LIP | 298.77±12.12 | 0.54±0.10 | 23.97±1.24 | 51.23±3.11 |

### 2. Comparison of PK parameters of IN administration and liposome

To investigate brain concentrations following IN administration and liposomal drug delivery, *in vivo* PK studies were performed in 2-month-old ICR mice, with comparison of the PK results to prior PK parameters of #2-NP.

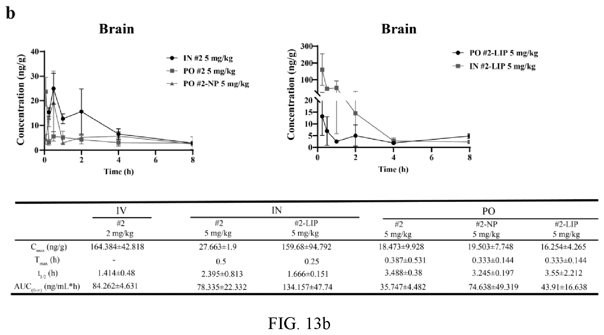

The results demonstrated that following IN administration of #2 at 5 mg/kg, the maximum plasma concentration (Cₘₐₓ) reached 947.291 ng/mL, which was approximately 10-fold higher than that observed with PO administration (90.809 ng/mL). However, the elimination half-life (t_{1/2}) was approximately half (1.542 h) that observed with PO administration (4.092 h). The mean residence time (MRT: 2.114 h) and clearance (CL: 2.637 L/h/kg) were both reduced compared to those with PO administration of #2. The AUC for IN administration was 1896.266 ng/mL*h, which substantially exceeded the AUC (405.15 ng/mL*h) for PO administration. Consequently, the bioavailability of #2 via IN administration increased to 34.3% from 6.99% (via PO administration) (FIG. 13a). Regarding the brain drug concentration, the maximum concentration levels of #2 were 18.47 ng/g for IV administration and 27.663 ng/g for PO administration, and the half-life levels in the brain were 3.49 h and 2.395 h, respectively. The AUCs for PO administration and IN administration were 35.75 ng/mL*h and 78.34 ng/mL*h, respectively, increasing by more than one-fold (FIG. 13b). Collectively, IN administration enhanced the bioavailability by more than four-fold and increased the brain exposure by more than one-fold compared to PO administration. Thus, IN administration is a better administration route for #2.

Similarly, the *in vivo* PKs of #2 following PO delivery of the liposome were evaluated. The results demonstrated that following PO administration of #2-LIP at identical doses (5 mg/kg), the maximum plasma concentration was 1,408.38 ng/mL, the half-life was 11.24 h, the area under curves was 2,996.664 ng/mL*h, the mean residence time was 17.75 h, and the clearance was 0.93; and all parameters were superior to those with PO administration of #2-NP (FIG. 13a). Consequently, the oral bioavailability of the liposome reached 51.76%. Regarding cerebral drug exposure in the brain, #2-LIP exhibited parameters comparable to #2-NP (FIG. 13b). This observation diverged from expectations. It is hypothesized that #2-NP enters the blood circulation system predominantly as a parent compound following PO administration, whereas #2-LIP may enter the blood circulation system in a liposome form, which does not facilitate BBB penetration of #2. Notably, this hypothesis requires further experimental validation.

Following IN administration, #2-LIP exhibited superior PK parameters and brain exposure. The results revealed that following IN administration of #2-LIP, the maximum plasma concentration reached 7,469.15 ng/mL, significantly higher than that (947.29 ng/mL) with IN administration of #2; and the time to maximum concentration was 0.25 h, shorter than 0.5 h observed with IN administration of #2, which might be caused by easier nasal mucosal absorption of #2-LIP. After IN #2-LIP, the elimination half-life was 1.254 h, the mean residence time was 1.386 h, and the clearance was 0.9, all of which were reduced as compared to IN #2. The area under the curves reached 5,609.404 ng/mL*h, and the bioavailability was 96.89% (FIG. 13a). Similarly, #2 in brain tissues was detected, and the results revealed that IN #2-LIP exhibited parameters comparable to IV #2 2 mg/kg: a maximum brain concentration of 159.68 ng/g, a time to maximum concentration of 0.25 h, an elimination half-life of 1.667 h, and an area under curves of 134.16 ng/mL*h (FIG. 13b).

Based on comprehensive comparative analysis of both formulations (nanoparticle vs. liposome) and administration routes (PO vs. IN), the present disclosure concludes that for #2, IN administration of the liposome is the best administration route. Subsequent PD studies will be conducted based on this conclusion.

It is obvious that the above examples are only instances for clear explanation, rather than limitation of the embodiments. For those of ordinary skill in the art, other different forms of changes or modifications may be made on the basis of the above descriptions. It is unnecessary and also unable to list all the possible embodiments. The obvious changes or modifications derived therefrom will still fall within the protection scope of the present disclosure.

## Claims

1. A CF3CN derivative, wherein the CF3CN derivative has any one of structural formulas 1-4 shown below:

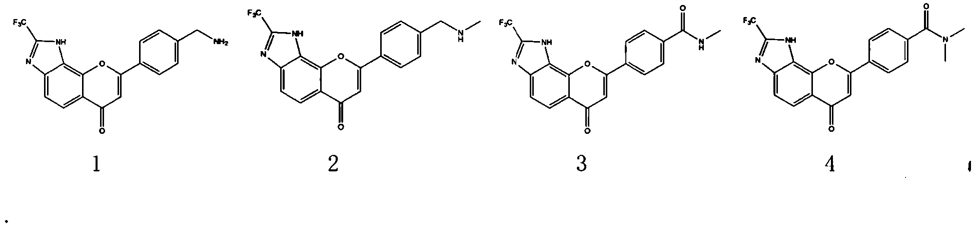

2. A pharmaceutical composition, wherein the pharmaceutical composition comprises one or more of CF3CN derivatives shown in formulas 1-4 below, and a pharmaceutically acceptable carrier or excipient, 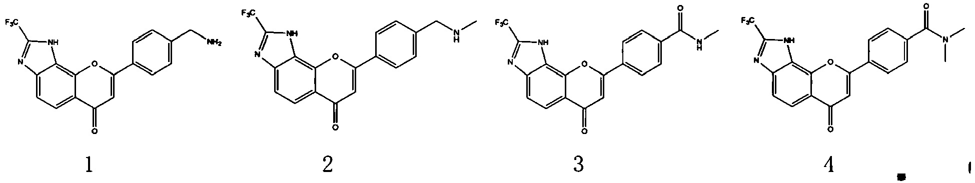 .

3. A nanoparticle, wherein the nanoparticle comprises one or more of CF3CN derivatives shown in formulas 1-4 below, and zein, and the nanoparticle is prepared by an antisolvent co-precipitation method,

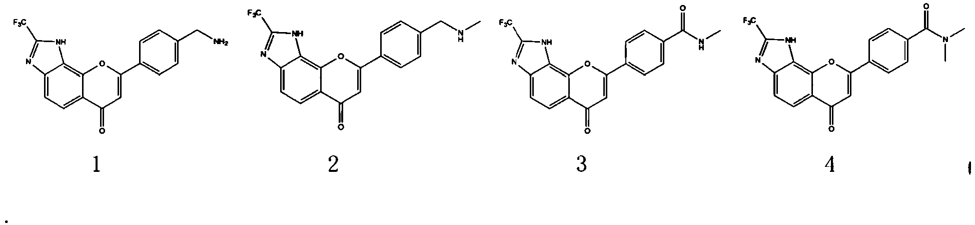

4. The nanoparticle according to claim 3, wherein the nanoparticle further comprises lactoferrin or glycosylated lactoferrin.

5. The nanoparticle according to claim 3, wherein a weight ratio of the CF3CN derivative to the zein is 1:1-1:20.

6. The nanoparticle according to claim 4, wherein a weight ratio of the CF3CN derivative to the zein to the lactoferrin or glycosylated lactoferrin is (1-2):(1-20):(1-20).

7. The nanoparticle according to claim 3, wherein the nanoparticle comprises a CF3CN derivative shown in the formula 2, zein, and lactoferrin, and a weight ratio of the CF3CN derivative shown in the formula 2 to the zein to the lactoferrin is (1-2):(1-20):(1-20).

8. The nanoparticle according to claim 3, wherein the nanoparticle is prepared by the antisolvent co-precipitation method comprising: dissolving one or more of the CF3CN derivatives shown in the formulas 1-4 and zein in a 70 vol% to 80 vol% aqueous ethanol solution to obtain a mixed solution,adding the mixed solution to deionized water under stirring, and evaporating the solvent to obtain the nanoparticle.

9. The nanoparticle according to claim 4, wherein the nanoparticle is prepared by the antisolvent co-precipitation method comprising: dissolving one or more of the CF3CN derivatives shown in the formulas 1-4, zein, and lactoferrin in a 70 vol% to 80 vol% aqueous ethanol solution to obtain a mixed solution,adding the mixed solution to deionized water under stirring, and evaporating the solvent to obtain the nanoparticle; or dissolving one or more of the CF3CN derivatives shown in the formulas 1-4 and zein in a 70 vol% to 80 vol% aqueous ethanol solution to obtain a mixed solution; dissolving glycosylated lactoferrin in an aqueous solution to obtain an aqueous glycosylated lactoferrin solution, and adding the mixed solution to the aqueous glycosylated lactoferrin solution under stirring, and evaporating the solvent to obtain the nanoparticle.

10. A liposome, wherein the liposome comprises one or more of CF3CN derivatives shown in formulas 1-4 below, and lecithin, and the liposome is prepared by an ethanol injection method: 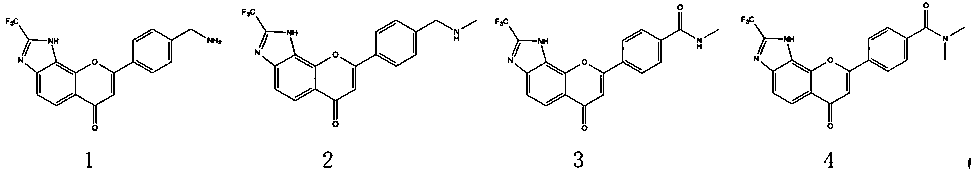 , and
a weight ratio of the lecithin to the CF3CN derivative is (1:1)-(50:1).

11. The liposome according to claim 10, wherein the liposome further comprises a stabilizing agent;
the stabilizing agent is selected from chitosan; and
a weight ratio of the lecithin to the chitosan to the CF3CN derivative is (1:1:1)-(50:1:1).

12. The liposome according to claim 10, wherein the liposome is prepared by the ethanol injection method comprising: dissolving one or more of CF3CN derivatives shown in the formulas 1-4 and lecithin in absolute ethyl alcohol to obtain a mixed solution,adding the mixed solution to deionized water under stirring, and evaporating the solvent to obtain a crude liposome solution; and
the method further comprises: subjecting the crude liposome solution to ice bath ultrasonication for 5-10 min; and dissolving chitosan in deionized water to obtain an aqueous chitosan solution, and adding the liposome solution to the aqueous chitosan solution under stirring to obtain the derivative-loaded liposome.

13. Applications of the CF3CN derivative according to claim 1 in preparing a TrkB agonist, or applications of the CF3CN derivative according to claim 1, the pharmaceutical composition according to claim 2, the nanoparticle according to claim 3, or the liposome according to claim 10 in preparing drugs for preventing and/or treating Alzheimer's disease (AD).

14. A method for preventing and/or treating AD, wherein the method comprises: administering to a subject a therapeutically effective amount of the CF3CN derivative according to claim 1, the pharmaceutical composition according to claim 2, the nanoparticle according to claim 3, or the liposome according to claim 10.

15. The method according to claim 14, an administration route is oral administration or intranasal administration.
